# EUROPÄISCHE PATENTSCHRIFT

(11) **EP 0 625 138 B1**
(45) Veröffentlichungstag und Bekanntmachung des Hinweises auf die Patenterteilung: **02.06.1999**
(21) Anmeldenummer: 93902195.2
(22) Anmeldetag: 14.01.1993
(51) Int. Cl.: C07C 257/18, A61K 31/155, C07D 311/22, C07D 213/78, A61K 31/35, A61K 31/44

(54) **NEUE AMIDINDERIVATE, IHRE HERSTELLUNG UND VERWENDUNG ALS ARZNEIMITTEL MIT LTB4-ANTAGONISTISCHER WIRKUNG**
NOVEL AMIDINE DERIVATIVES, THEIR PREPARATION AND THEIR USE AS MEDICAMENTS WITH LTB4 ANTAGONISTIC EFFECT
NOUVEAUX DERIVES DE L'AMIDINE, LEUR FABRICATION ET LEUR APPLICATION EN TANT QUE MEDICAMENTS AYANT UN EFFET ANTAGONISTE A LTB4

(30) Priorität: 05.02.1992 DE 4203201; 23.07.1992 DE 4224289; 24.12.1992 DE 4244241
(43) Veröffentlichungstag der Anmeldung: 23.11.1994
(60) Teilanmeldung: 98121305.1
(73) Patentinhaber: BOEHRINGER INGELHEIM INTERNATIONAL GmbH, 55218 Ingelheim am Rhein (DE); Boehringer Ingelheim Pharma KG, 55216 Ingelheim am Rhein (DE)
(72) Erfinder: ANDERSKEWITZ, Ralf, D-6530 Bingerbrück (DE); SCHROMM, Kurt, D-6507 Ingelheim (DE); RENTH, Ernst-Otto, D-6507 Ingelheim (DE); HIMMELSBACH, Frank, D-7951 Mittelbiberach (DE); BIRKE, Franz, D-6507 Ingelheim (DE); FÜGNER, Armin, D-6535 Gau-Algesheim (DE)
(74) Vertreter: Laudien, Dieter, Dr.
(86) Internationale Anmeldenummer: EP9300070
(87) Internationale Veröffentlichungsnummer: WO9316036

(56) Entgegenhaltungen:
- EP-A- 0 292 977
- EP-A- 0 366 066
- EP-A- 0 518 818
- EP-A- 0 518 819
- JOURNAL OF MEDICINAL CHEMISTRY Bd. 12, 1969, WASHINGTON US Seiten 408 - 414 B.R.BAKER ET AL. 'IRREVERSIBLE ENZYME INHIBITORS. CLII. PROTEOLYTIC ENZYMES. X. INHIBITION OF GUINEA PIG COMPLEMENT BY SUBSTITUTED BENZAMIDINES'
- JOURNAL OF MEDICINAL CHEMISTRY Bd. 17, Nr. 11, 1974, WASHINGTON US Seiten 1160 - 1166 C. HANSCH ET AL. 'STRUCTURE-ACTIVITY RELATIONSHIP IN IMMUNOCHEMISTRY.2. INHIBITION OF COMPLEMENT BY BENZAMIDES.'

## Beschreibung

Die Erfindung betrifft neue Amidinderivate, ihre Herstellung nach konventionellen Methoden und ihre Verwendung in Arzneimitteln.

Die erfindungsgemäßen Verbindungen sind therapeutisch verwendbar, insbesondere aufgrund ihrer LTB₄-antagonistischen Wirkung. Aus der Europäischen Patentanmeldung EP 0 292 977 (30. November 1988) sind Verbindungen mit LTB₄-antagonistischer Wirkung bekannt. Während die vorliegende Erfindung neue Amidin-Derivate mit LTB4-antagonistischer Wirkung offenbart, zielt die EP 0 292 977 auf Alkoxy-substituierte Dihydropyran-2-carbonsäurederivate. Aus J. Med. Chem. 17, 1160-1166 (1974) sind pharmakologisch interessante Benzamidine bekannt. Die dort offenbarten Verbindungen zeigen eine Wirksamkeit bei der Inhibition des Meerschweinchen Komplementsystems. Die durch die vorveröffentlichten Europäischen Patentanmeldungen EP 0 518 818 und EP 0 518 819 (beide 16 Dezember 1992) offenbarten Benzamidin-Derivate weisen wie die erfindungsgemäßen Verbindungen LTB₄-antagonistische Wirksamkeit auf.

Die neuen Amidinderivate entsprechen der Formel 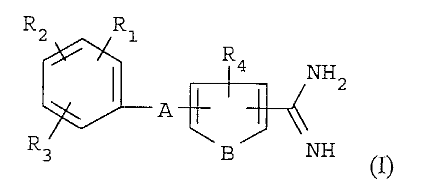 in der
- R₁ und R₂,: die gleich oder verschieden sein können, für CF₃, Halogen, R₅, OR₅, COR₆, SR₆, SOR₆, SO₂R₆, SO₂NR₅R₇, C(OH)R₅R₇ oder gemeinsam auch für die mit benachbarten C-Atomen des Benzolrings verknüpften zweibindigen Gruppen -CH=CH-CH=CH-, -CR₈=CH-CH=CH-,-CH=CR₈-CH=CH-, -O-CH₂-CH₂, -O-CH₂-O-, -O-CH₂-CH₂-O-, - CH₂)₃₋₄-, -NH-CO-O-, -NH-CO-CH₂-O-, -CO-CH₂-O- oder -CO-CH₂CH₂-O- stehen, wobei diese Gruppen ihrerseits auch durch C₁-C₄-Alkyl substituiert sein können,
- R₃: für Halogen, OH, CF₃, R₅, OR₆, COR₆, SR₆, SOR₆, SO₂R₆, SO₂NR₅R₇, NR₅R₇ oder C(OH)R₅R₇ steht (wobei, falls R₃ gleich R₅ ist, R₅ nur dann H sein kann, wenn mindestens einer der Substituenten R₁ und R₂ nicht H bedeutet),
- R₄: für Wasserstoff,Halogen, OH, C₁-C₄-Alkoxy,
- R₅: für H, C₁-C₁₂-Alkyl, Phenyl, gegebenenfalls durch Halogen, C₁-C₄-Alkyl, C₁-C₄-Alkoxy oder C₂-C₅-Acyl substituiertes Phenyl,
- R₆: für C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
- R₇: für H oder C₁-C₁₂-Alkyl,
- R₈: C₂-C₅-Acyl,
- A: für eine der Gruppen

X₁ -A₁-X₂ (II)

X₂-A₂-X₃ (III)

X₄-A₂-X₂ (IV)

(CH₂)₁₋₂-NH-CO-(CH₂)₁₋₃-X₂ (V)

-CH=CH-A₂-X₂ (VI)
- B: für CH=CH, CH=N, S oder 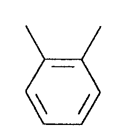
- A₁: für C₂-C₄-Alkylen, cis- oder trans-CH₂-CH=CH-CH₂, CH₂-C≡C-CH₂ oder 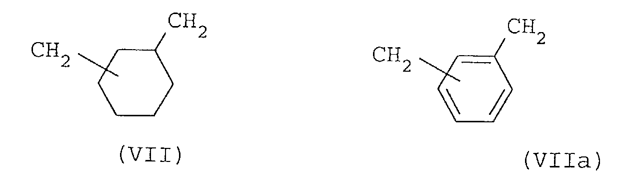
- A₂: für C₁-C₅-Alkylen,
- X₁: für O, NH, S, SO, SO₂, CO oder CH₂,
- X₂: für O, NH oder S,
- X₃: für NH-CO, CO-NH oder SO₂-NM,
- X₄: für NH-CO, CO-NH, NH-SO₂ oder SO₂-NH steht,
mit der Maßgabe, daß falls
- B: -CH=CH- und
- X₁: Sauerstoff oder Schwefel und
- X₂: Sauerstoff oder Schwefel und
- A₁: C₂-C₄-Alkylen, cis- oder trans-CH₂-CH=CH-CH₂, CH₂-C≡C-CH₂ oder 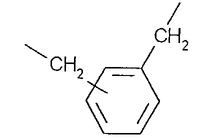 und einer der Reste R₁, R₂ oder R₃ -O-C₁-C₁₂-Alkyl bedeuten,
mindestens einer der anderen Reste R₁, R₂ oder R₃ nicht
Wasserstoff, Halogen, CF₃, OH, C₁-C₁₂-Alkyl oder -O-C₁-C₁₂-Alkyl sein kann,
sowie mit der Maßgabe, daß falls
- B: -CH=CH-
- R₄: Wasserstoff,
- X₁ und X₂: Sauerstoff,
- A₁: C₃- oder C₄-Alkylen
bedeuten und die Amidino-Gruppe zum Rest A in meta-Position steht,
R₁, R₂ oder R₃ nicht Phenyl, NH₂, Chlor, Methoxy oder CF₃ bedeuten können,
als Racemate, in enantiomerenreiner bzw. angereicherter Form, gegebenenfalls als Diastereomerenpaare sowie in cis- oder trans-Form und jeweils als freie Basen oder als Salze, vorzugsweise mit physiologisch verträglichen Säuren.

Bevorzugt sind (im Rahmen der obigen Definitionen) Verbindungen der Formel 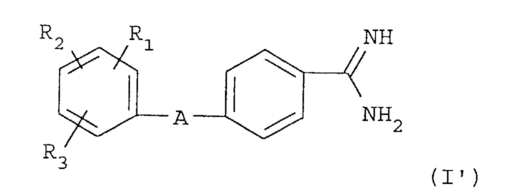 in der
- R₁, R₂,: die gleich oder verschieden sein können, R₇, OR₇, COR₆ oder gemeinsam auch die mit benachbarten C-Atomen des Benzolrings verknüpften zweibindigen Gruppen -CR₈=CH-CH=CH-, -CH=CR₈-CH=CH-, -O-CH₂-CH₂- oder -CO-CH₂-CH₂-O- bedeuten, wobei diese Gruppen ihrerseits auch durch C₁-C₄-Alkyl substituiert sein können,
- R₃: für Halogen, OH, CF₃, R₅, OR₆, COR₆, SR₆, SOR₆, SO₂R₆, SO₂NR₅R₇,NR₅R₇ oder C(OH)R₅R₇ steht (wobei, falls R₃ gleich R₅ ist, R₅ nur dann H sein kann, wenn mindestens einer der Substituenten R₁ und R₂ nicht H bedeutet),
- R₅: für H, C₁-C₁₂-Alkyl, Phenyl, gegebenenfalls durch Halogen, C₁-C₄-Alkyl, C₁-C₄-Alkoxy oder C₂-C₅-Acyl substituiertes Phenyl,
- R₆: für C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
- R₇: für H oder C₁-C₁₂-Alkyl,
- R₈: für C₂-C₅-Acyl,
und
- A: für die Gruppe X₁-A₁-X₂ (II) steht, wobei
- A₁: C₂-C₄-Alkylen, cis- oder trans-CH₂-CH=CH-CH₂, CH₂-C≡C-CH₂ oder 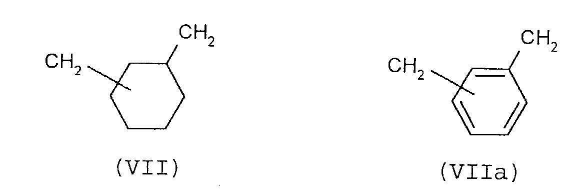
- X₁: O, NH, S, SO, SO₂, CO oder CH₂,
- X₂: O, NH oder S bedeutet,
mit der Maßgabe, daß falls
- X₁: Sauerstoff oder Schwefel und
- X₂: Sauerstoff oder Schwefel und
- A₁: C₂-C₄-Alkylen, cis- oder trans-CH₂-CH=CH-CH₂, CH₂-C≡C-CH₂ oder 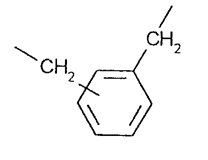 und
einer der Reste R₁, R₂ oder R₃ -O-C₁-C₁₂-Alkyl bedeuten,
mindestens keiner der anderen Reste R₁, R₂ oder R₃ nicht Wasserstoff, Halogen, CF₃, OH, C₁-C₁₂-Alkyl oder -O-C₁-C₁₂-Alkyl sein kann,
als Racemate, in enantiomerenreiner bzw. angereicherter Form, gegebenenfalls als Diastereomerenpaare sowie in cis- oder trans-Form und jeweils als freie Basen oder als Salze, vorzugsweise mit physiologisch verträglichen Säuren.

Als Gruppe der Formel -C₆H₂R₁R₂R₃ seien besonders hervorgehoben:

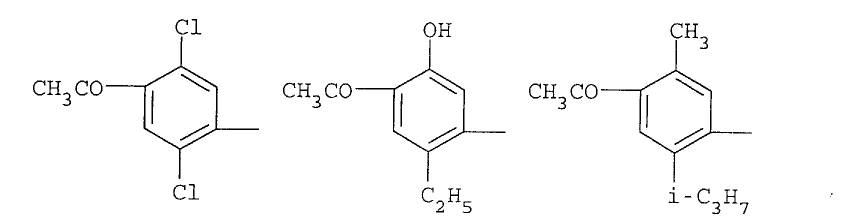

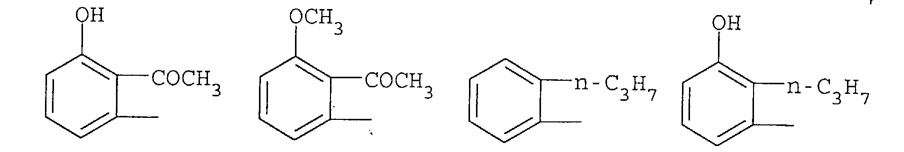

Unter den Bedeutungen von A seien hervorgehoben:

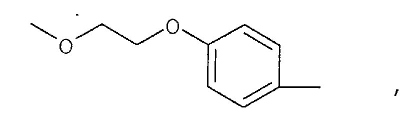

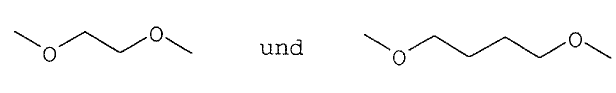

In den obigen Definitionen bedeutet "Halogen" F, Cl, Br oder J, vorzugsweise F, Cl. Soweit die aufgeführten Gruppen Alkylketten sind oder solche enthalten, können diese geradkettig oder verzweigt sein. Die Alkylketten in R₅, R₆ und R₇ enthalten bevorzugt bis zu 6 C-Atome, vor allem 1 bis 4 C-Atome. Insbesondere als Bestandteil von COR₆ kann R₆ in der Bedeutung "Alkyl" auch ein- oder mehrfach fluorsubstituiert sein. Als Substituenten von Ringsystemen sind als Alkyle Methyl, Ethyl und die Propyle hervorzuheben. Ein bevorzugter Acylrest ist COCH₃, bevorzugter Alkoxyrest ist CH₃O. Die Brücke A enthält bevorzugt 4 bis 6 Glieder. Die Gruppe ist so zwischen den beiden Ringsystemen in Formel I und in entsprechenden Formeln angeordnet, wie es der Schreibung der Formeln II bis VI entspricht, während die für R₁ und R₂ gemeinsam geltenden Gruppen nicht richtungsorientiert aufgeführt sind. Bedeutet R₁ und R₂ gemeinsam eine zweibindige Gruppe, so steht R₃ vorzugsweise für H oder C₂-C₅-Acyl, etwa Acetyl. Die Gruppen R₁, R₂ und R₃ sollen nicht alle gleichzeitig für CF₃, COR₆, SR₆, SOR₆, SO₂R₆, SO₂NR₅R₇ oder C(OH)R₅R₇ stehen, vielmehr sind diese Gruppen sowie OR₅ in der Bedeutung Phenoxy bzw. substituiertes Phenoxy bevorzugt nur einmal oder eventuell auch zweimal vorhanden, wobei als weitere Substituenten vor allem Alkyl, Acyl, Halogen hinzutreten können. Die Bindungen bzw. CH₂-Gruppen in VII/VIIa befinden sich im allgemeinen in α-Stellung zueinander. Typische Gruppen für A sind z.B. O-(CH₂)₂-O, O-(CH₂)₄-O, wobei auch eines der O-Atome durch S, NH oder CO ersetzt sein kann, ferner Gruppen wie CH₂-CH₂-CONH, CH₂-CH₂-NH-CO, CO-NH-CH₂-CH₂ oder NH-CO-CH₂-CH₂. Die Amidinogruppe befindet sich meist in para-Stellung zu dem C-Atom, mit dem A verknüpft ist.

Die neuen Verbindungen werden nach konventionellen Methoden hergestellt.
1. Umsetzung von Imidoestern der Formel 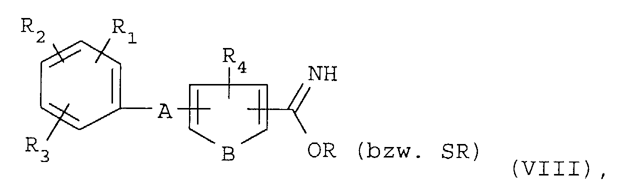 in der R₁ bis R₄, A und B die obige Bedeutung haben und R bevorzugt für einen C₁-C₆-Alkylrest oder für Benzyl steht (jedoch kann der Fachmann gewünschtenfalls auch Derivate anderer Alkohole einsetzen), und Ammoniak. Die Umsetzung erfolgt zweckmäßig in einem organischen Lösungsmittel bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist. Geeignete Lösungsmittel sind polare Lösungsmittel wie Methanol, Ethanol, Propanole.
   Bei hinreichend säurestabilen Ausgangsstoffen kann die Umsetzung statt über die Imidoester auch über die entsprechenden Säureimidchloride erfolgen.
2. Zur Herstellung von Verbindungen der Formel I, in denen A eine über O oder S mit mindestens einem der Ringsysteme verknüpft ist:
   Umsetzung
   (a) eines Phenols oder Thiophenols der Formel 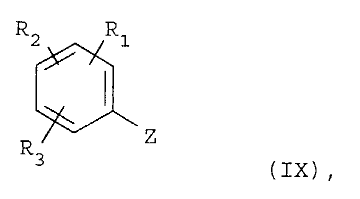 worin Z OH oder SH darstellt und R₁, R₂ und R₃ die obige Bedeutung haben, mit einer Verbindung der Formel 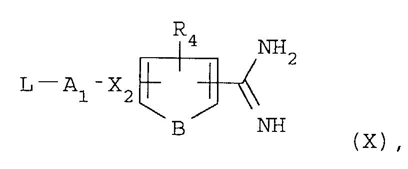 bzw. 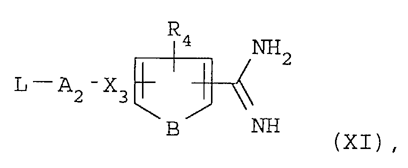 worin A₁, A₂, B, R₄, X₂ und X₃ die obige Bedeutung haben und L für eine nucleofuge Abgangsgruppe steht, bzw.
   (b) eines Phenols oder Thiophenols der Formel 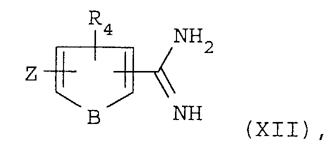 worin B, R₄ und Z die obige Bedeutung haben, mit einer Verbindung der Formel 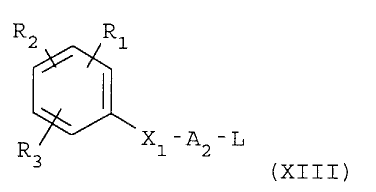 bzw. 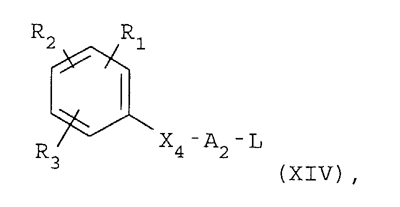 bzw. 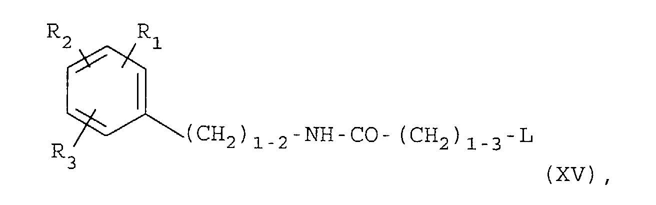 bzw. 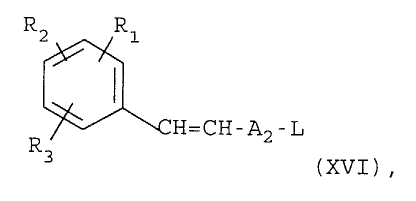 worin A₁, A₂, R₁, R₂, R₃ und L die obige Bedeutung haben.

   Die Umsetzung erfolgt in aprotischen Lösungsmitteln wie Dimethylsulfoxid, Dimethylformamid, Acetonitril oder Alkoholen wie Methanol, Ethanol oder Propanol unter Zusatz einer Base (Metallcarbonate,
   Metallhydroxide, Metallhydride) bei Temperaturen zwischen etwa 0 und 140°C bzw. der Siedetemperatur des Reaktionsgemischs.
   Die Phenole oder Thiophenole können auch in Form von Salzen, etwa der Alkalisalze, eingesetzt werden. Als nucleofuge Abgangsgruppe eignen sich z.B. Halogene, etwa Br, Cl.
3. Reduktion eines Amidoxims der Formel 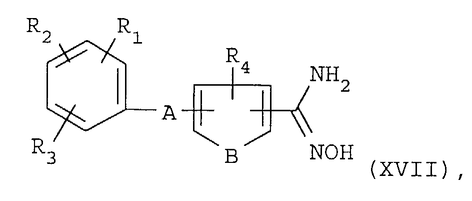 worin A, B und R₁ bis R₄ die obige Bedeutung haben.
   Für die Stufe der Reduktion von XVII eignet sich die katalytische Hydrierung, insbesondere mit Raney-Nickel in einem niederen Alkohol, z.B. Methanol.
   Zweckmäßig wird das Amidoxim der Formel XVII unter Zugabe der berechneten Menge derjenigen Säure, deren Salz als Endprodukt gewünscht wird, in Methanol gelöst und bei Raumtemperatur unter leichtem Druck, z.B. bei 5 bar, bis zur beendeten Wasserstoffaufnahme hydriert.

Die Ausgangsstoffe können nach üblichen Methoden aus bekannten Verbindungen erhalten werden.

So können die Ausgangsstoffe für Verfahren 1 aus den entsprechenden Nitrilen durch Umsetzung mit HCl über die Stufe der Imidchloride bzw. direkt durch Umsetzung mit z.B. C₁-C₆-Alkoholen bzw. Benzylalkohol in Gegenwart einer Säure wie HCl erhalten werden. Auch die Umsetzung der Nitrile mit H₂S in Lösungsmitteln wie Pyridin oder Dimethylformamid in Gegenwart einer Base wie Triethylamin und anschließende Alkylierung bzw. Benzylierung führen zu Verbindungen der Formel VIII. Ausgehend von Carbonsäureamiden, die im übrigen den Verbindungen der Formel VIII entsprechen, gelangt man auch durch Umsetzung mit einem Trialkyloxoniumsalz wie Triethyloxoniumtetrafluoroborat in einem Lösungsmittel wie Dichlormethan, Tetrahydrofuran oder Dioxan bei Temperaturen zwischen 0 und 50°C, vorzugsweise bei Raumtemperatur zu Verbindungen der Formel VIII.

Für die Herstellung der Ausgangsstoffe XVII können auch Umsetzungen entsprechender Amidoxime anstelle von Amidinen analog Verfahren 1 oder 2 dienen; durch analoge oder Umsetzung entsprechender Nitrile, aus denen abschließend durch Addition von Hydroxylamin die Ausgangsstoffe XVII entstehen.

Die erfindungsgemäßen Verbindungen sind therapeutisch verwendbar, insbesondere aufgrund ihrer LTB4-antagonistischen Wirkung. Sie eignen sich daher für die Anwendung vor allem bei solchen Krankheiten, bei denen entzündliche und/oder allergische Vorgänge eine Rolle spielen, beispielsweise, Asthma, Colitis ulcerosa, Psoriasis, ferner zur Behandlung einer durch nichtsteroidale Antiphlogistika induzierten Gastropathie. Die neuen Verbindungen können auch in Kombination mit anderen Wirkstoffen angewendet werden, z. B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 250 mg, vorzugsweise zwischen 20 und 200 mg. Bei inhalativer Anwendung werden dem Patienten zwischen etwa 2 und 20 mg Wirkstoff zugeführt. Die neuen Verbindungen können in üblichen Zubereitungen verabreicht werden, etwa als Tabletten, Dragees, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen.

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 3 345 722, inhaliert.

Die erfindungsgemäßen Verbindungen wurden u.a. auf ihre Wirkung in den nachstehenden Testen untersucht.
a) U937 - Rezeptorverbindungstest/LTB₄
   Die Bindung von ³H-LTB₄ (3nM) auf vitalen U937-Zellen (differenzierte, humane monozytäre Zellinie mit natürlich exprimierten LTB₄-Rezeptoren) wird durch steigende Konzentration der Testsubstanz dosisabhängig inhibiert (Inkubation 2h bei 0°C). Nach Abtrennung des ungebundenen ³H-LTB₄ durch Membranfiltration wird die Radioaktivität des gebundenen LTB₄-Rezeptor/³H-LTB₄-Komplexes durch Szintilationsmessung quantifiziert. Die Bestimmung der Affinität (Inhibitionskonstante Kᵢ) erfolgte durch iterative Anpassung einer Verdrängungskurve an die Meßwerte (Programm: "gekoppelte Massengleichgewichte" auf Wang-Computer).
b) Aggregation von neutrophilen Granulozyten des Meerschweinchens
   Indiziert durch LTB₄ in vitro (Zunahme der Lichttransmission im Aggregometer, aufgezeichnet in mm; je Experiment Doppelbestimmung): Hemmung 2 min nach Inkubation mit Prüfsubstanz in Polydiol/DMSO.
c) Leukotrien-B₄-indizierte Neutrophilen-Akkumulation am Mäuseohr
   Bewertung des neutrophilen Einstroms durch fotometrische Messung (mOD/min) der Myeloperoxidaseaktivität (Bradley et al.: J. Invest. Dermatol. 78, 206, 1982) in der Ohrhaut. Zunahme 6h nach topischer Behandlung des linken Ohres mit LTB₄ (beidseitig je 250 ng) gegenüber dem rechten Ohr (2 x 5 µl Aceton als Lösungsmittel).
   Substanzgabe per os in 1 %iger Tylose 300, 30 min vor LTB₄-Reiz.
(4) Ergebnisse 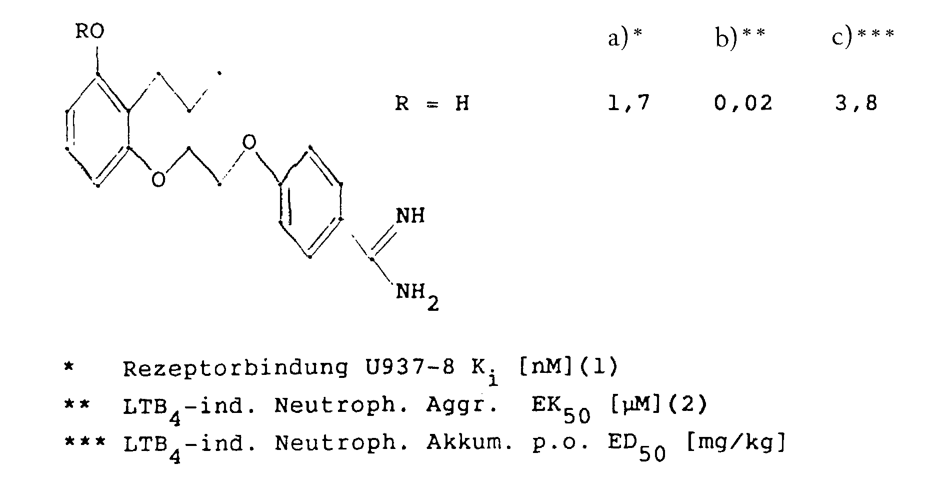
   Die ³H-LTB₄-Rezeptorbindung an MeerschweinchenMilzzellen in Gegenwart von 10 % Blutplasma lieferte Kᵢ-Werte von z.T. weit unter 1 µM, insbesondere zwischen 0,2 und 0,02. Die Hemmung der LTB₄-induzierten Neutrophilen-Aggregation ergab EC₅₀-Werte zwischen etwa 0,5 und 0,05 µM.
   Hervorzuheben sind die Verbindungen nach Beispiel 1 sowie Nr. 8, 9, 11, 17, 18, 20, 21 aus Tabelle I, Nr. 1 aus Tabelle II, Nr. 2 aus Tabelle III.
   Die nachstehenden Beispiele verdeutlichen die Herstellmöglichkeiten der erfindungsgemäßen Verbindungen.

### Verfahren 1:

### Beispiel 1

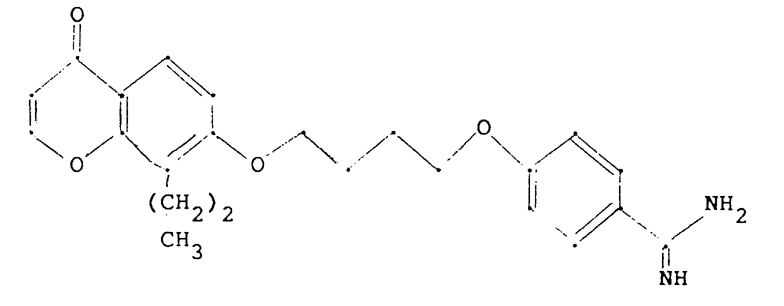

Zu einer Lösung von 2,0 g 7-[4-(4-Cyano-phenoxy)-E-but(2)-enyloxy]-8-propyl-4H-1-benzopyran-4-on in 50 ml Chloroform und 1,5 ml Ethanol gibt man 5 ml einer Lösung von Chlorwasserstoff in Diethylether (17 %). Man läßt das Gemisch 14 Tage bei Raumtemperatur stehen und fällt das Produkt mit Diethylether. Man erhält 1,15 g 7-[4-(4-Imidacarboxyethyl-phenoxy)-E-but(2)-enyloxy]-8-propyl-4H-1-benzopyran-4-on-hydrochlorid. Der Imidoester wird mit 50 ml ethanolischer Ammoniaklösung (5 M) versetzt und 3 Stunden bei 70°C erwärmt. Man dampft das Gemisch ein und chromatographiert den Rückstand (Chloroform/Methanol 7:3, Kieselgel). Nach Umkristallisieren aus Dichlormethan/Diethylether erhält man 0,6 g 7-[4-(4-Amidino-phenoxy)-E-but(2)-enyloxy]-8-propyl-4H-1-benzopyran-4-on-hydrochlorid (Fp. 144 - 148°C).

### Beispiel 2

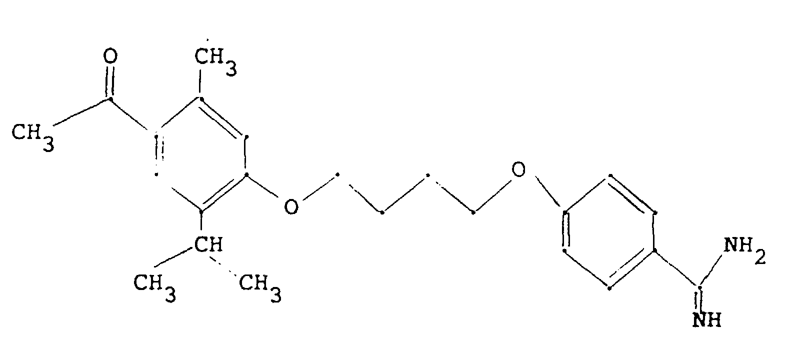

In eine Lösung von 32,0 g 4-[(4-Acetyl-2-isopropyl-5-methyl-phenoxy)-butyloxy]-benzonitril in 350 ml Ethanol leitet man bei -20°C Chlorwasserstoff ein und rührt 48 Stunden nach. Die ausgefallenen Kristalle werden abgesaugt und mit Diethylether gewaschen. Man erhält 41,0 g 4-[4-(4-Acetyl-2-isopropyl-5-methylphenoxy)-butyloxy]-benzimidoethylester-hydrochlorid (Fp. 100 - 102°C Zers.). 15,0 g des Imidoesters werden bei Raumtemperatur in mehreren Portionen zu 33 ml ethanolischer Ammoniaklösung (5 M) und 100 ml Ethanol gegeben. Man läßt das Gemisch 36 Stunden bei Raumtemperatur rühren, dampft das Gemisch ein und verrührt den Rückstand mit 50 ml Wasser. Man saugt den Rückstand ab, kristallisiert aus 30 ml Ethanol um und wäscht mit Diethylether nach. Man erhält 11,5 g 4-[4-(4-Acetyl-2-isopropyl-5-methyl-phenoxy)-butyloxy]benzamidin-hydrochlorid (Fp. 182 - 183°C Zers.).

### Beispiel 3

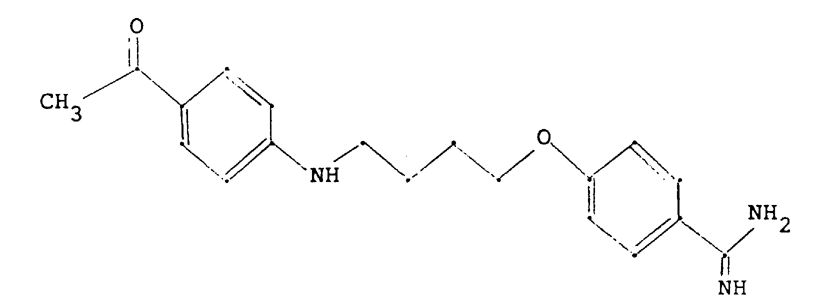

In eine Lösung von 3,0 g 4-[4-(4-Cyano-phenoxy)-butylamino]-acetophenon in 40 ml Ethanol leitet man bei -20°C unter Rühren 4 Stunden lang Chlorwasserstoff ein und läßt das Gemisch 16 Stunden bei Raumtemperatur stehen. Man destilliert das Lösungsmittel im Vakuum ab und nimmt den Rückstand in 50 ml Ethanol auf. Dazu tropft man ein Gemisch aus 14 ml ethanolischer Ammoniaklösung und 50 ml Ethanol und läßt das Gemisch 24 Stunden bei Raumtemperatur stehen. Das Lösungsmittel dampft man ab und chromatographiert den Rückstand (Chloroform/Methanol 7:3, Kieselgel 60). Man erhält 0,3 g 4-[4-(4-Amidinophenoxy)butylamino]-acetophenon (Fp. 200 - 202°C).

### Verfahren 2:

### Beispiel 4

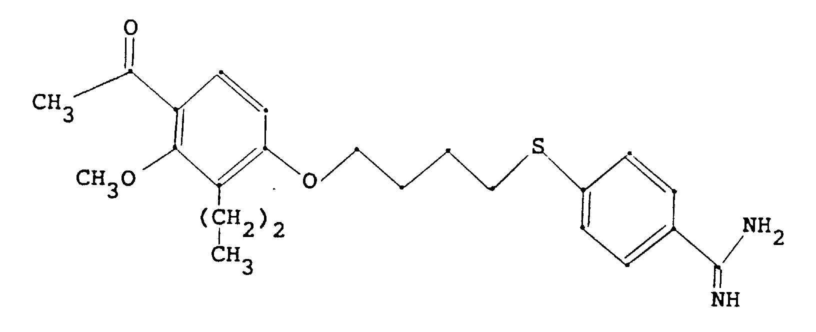

Man löst 8,2 g 4-Acetyl-3-methoxy-2-propyl-phenol in 80 ml Dimethylformamid und versetzt die Lösung portionsweise mit 1,1 g Natriumhydrid (als 80 proz. Dispersion in Weissöl). Man erwärmt das Gemisch 30 Minuten bei 80°C und versetzt mit einer Lösung von 5,75 g 4-(4-Brompropylthio)-benzamidin (hergestellt aus Dibrombutan und 4-Cyanobenzothiol über 4-(4-Brombutyl-thio)-benzonitril) in 40 ml Dimethylformamid. Nach 5 Stunden bei 80°C läßt man abkühlen, säuert mit etherischer Salzsäure an und destilliert die Lösungsmittel im Vakuum ab. Man nimmt den Rückstand in Ethanol auf und filtriert. Das Filtrat wird eingeengt. Man wiederholt den Vorgang mit Chloroform und Acetonitril. Der Rückstand wird mit Diethylether verrührt. Nach dem Abdekantieren verbleiben 5,65 g eines gelbbraunen Öls. Das Produkt wird chromatographiert (Chloroform/Methanol 7:3, Kieselgel). Man erhält 2,4 g eines Öls, das aus Toluol kristallisiert wird. Man löst das Produkt in Acetonitril, säuert mit etherischer Salzsäure an. Die Kristalle werden abgesaugt, mit kaltem Acetonitril gewaschen, in Wasser gelöst und nach Zugabe von 2 N Salzsäure nochmals kristallisiert. Man erhält 0,8 g 4-[4-(4-Acetyl-3-methoxy-2-propylphenoxy)-butylthiolbenzamidin-hydrochlorid (Fp. 120 - 122°C).

### Verfahren 3:

### Beispiel 5

a) 4-[4-(4-Acetylphenoxy)-butoxy]-benzamidoxim
   In 300 ml Dimethylformamid werden 45,6 g (0,3 mol) 4-Hydroxybenzamidoxim und 81,3 g (0,3 mol) 4-Brombutoxy-acetophenon gelöst. Nach Zugabe von 55,2 g (0,4 mol) wasserfreiem Kaliumcarbonat wird 2 Stunden auf 80°C erwärmt. Man saugt die anorganischen Salze ab, engt i.V. ein und kristallisiert aus Acetonitril um.
   Ausbeute: 47,8 g
   Fp.: 164,5 - 165,5°C.
b) 3-[4-(4-Acetylphenoxy)butoxy]-benzamidin - Methansulfonat
   47,8 g der nach a) synthetisierten Verbindung werden in der 10fachen Menge Methanol unter Zugabe der berechneten Menge Methansulfonsäure gelöst. Nach Zugabe von Raney-Nickel wird bei 5 bar bis zur beendeten Wasserstoffaufnahme hydriert. Man saugt ab, destilliert das Lösungsmittel i.V. ab und kristallisiert aus Ethanol um.
   Ausbeute: 45,2 g
   Fp.: 204 - 204,5°C.

Entsprechend den obigen Verfahren können die weiteren Verbindungen der Formel I erhalten werden. "Ac" bedeutet im folgenden CH₃CO-.

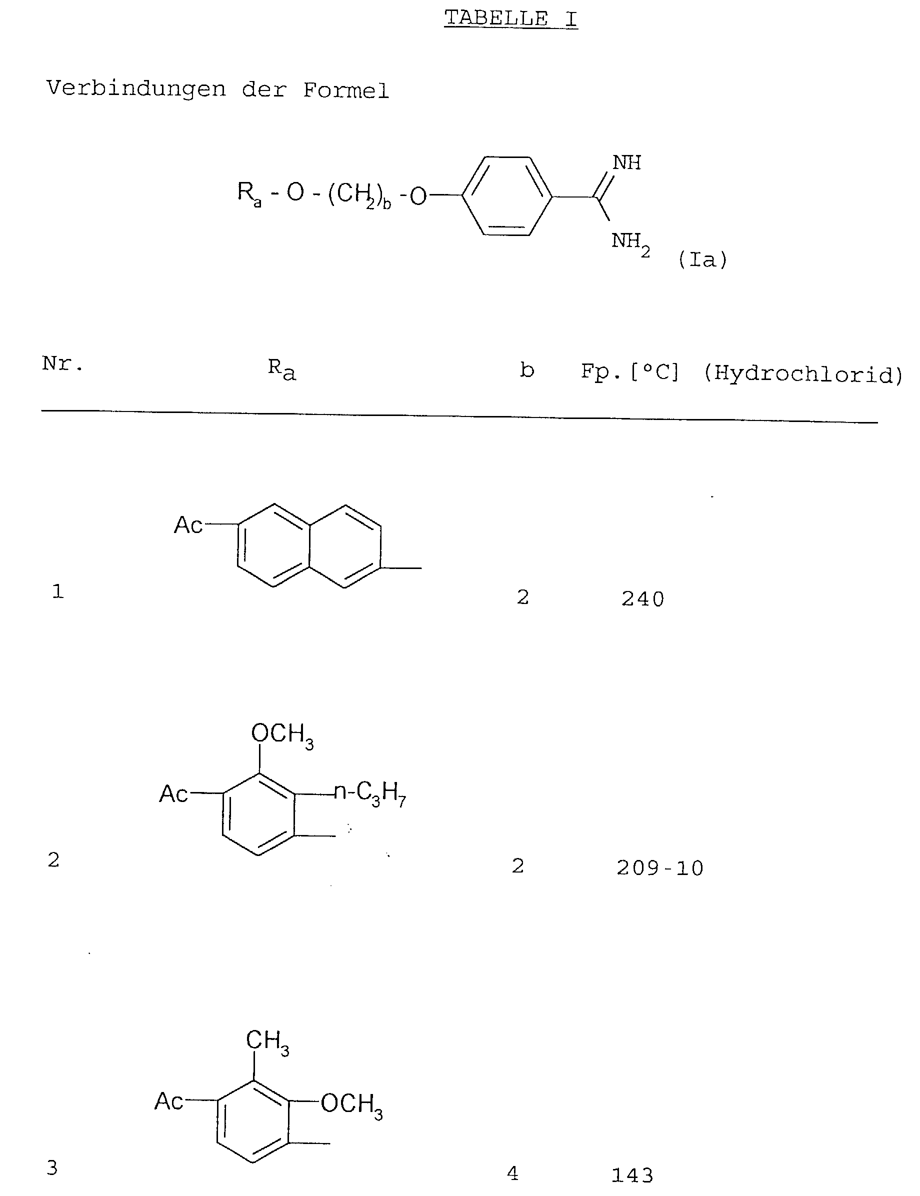

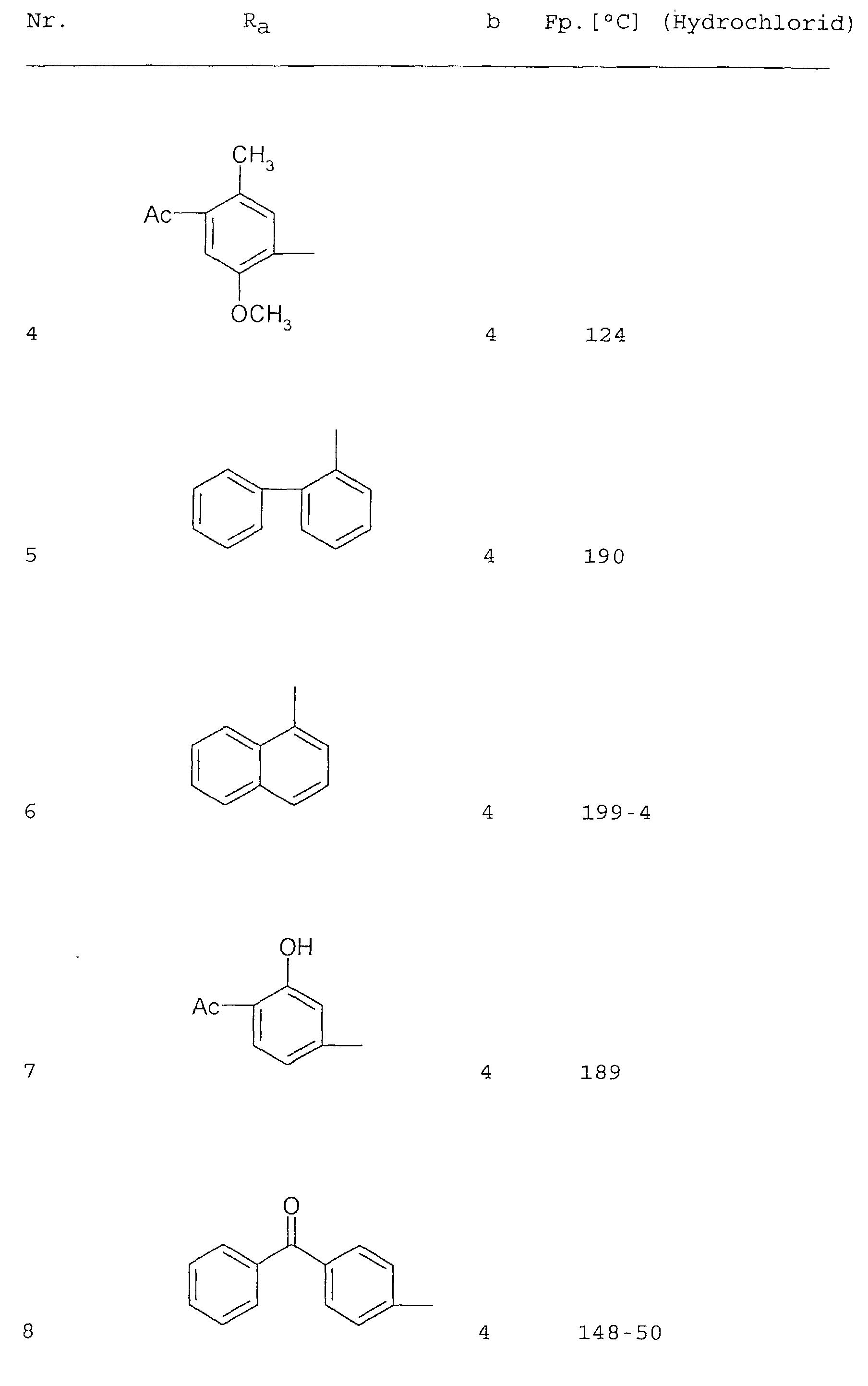

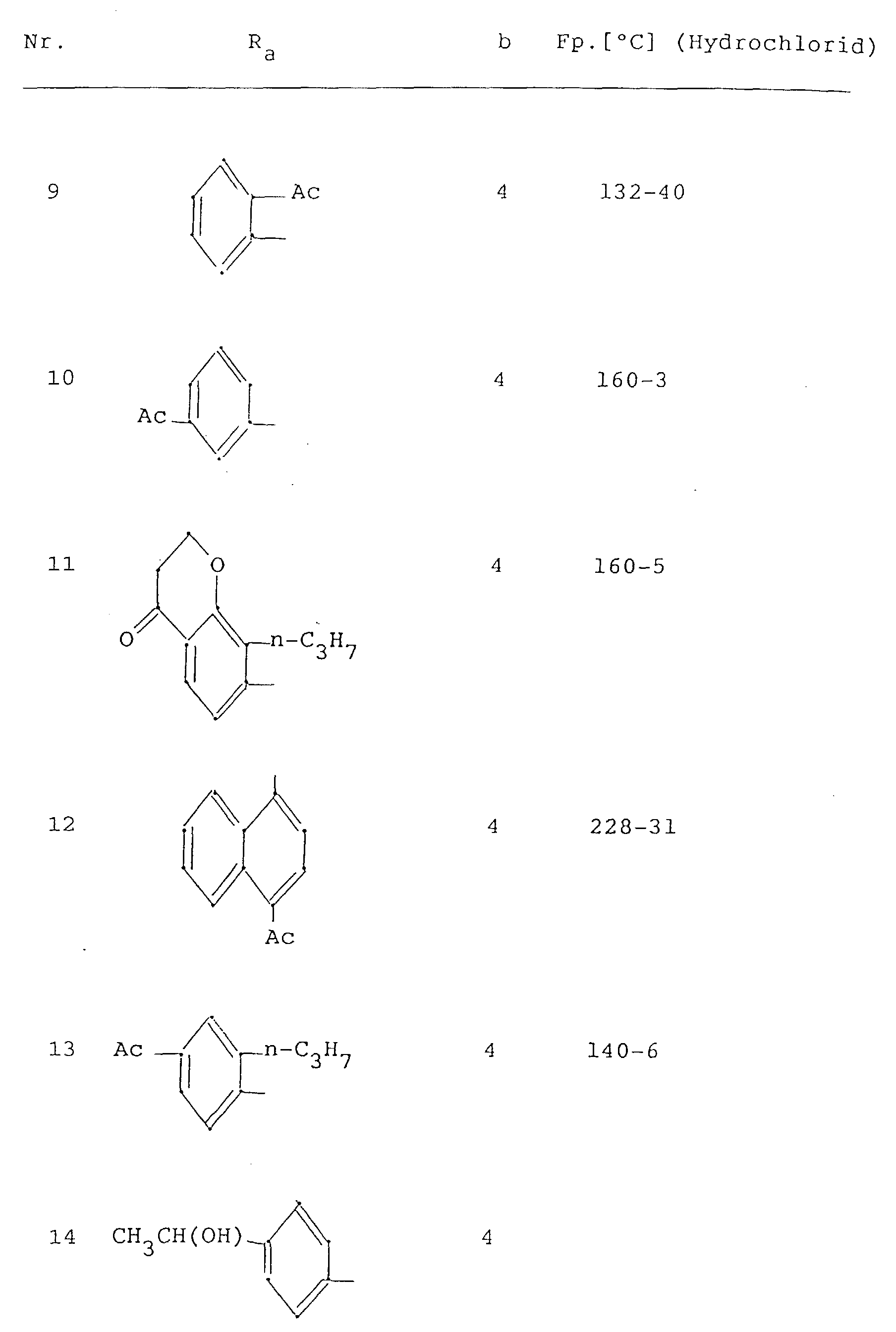

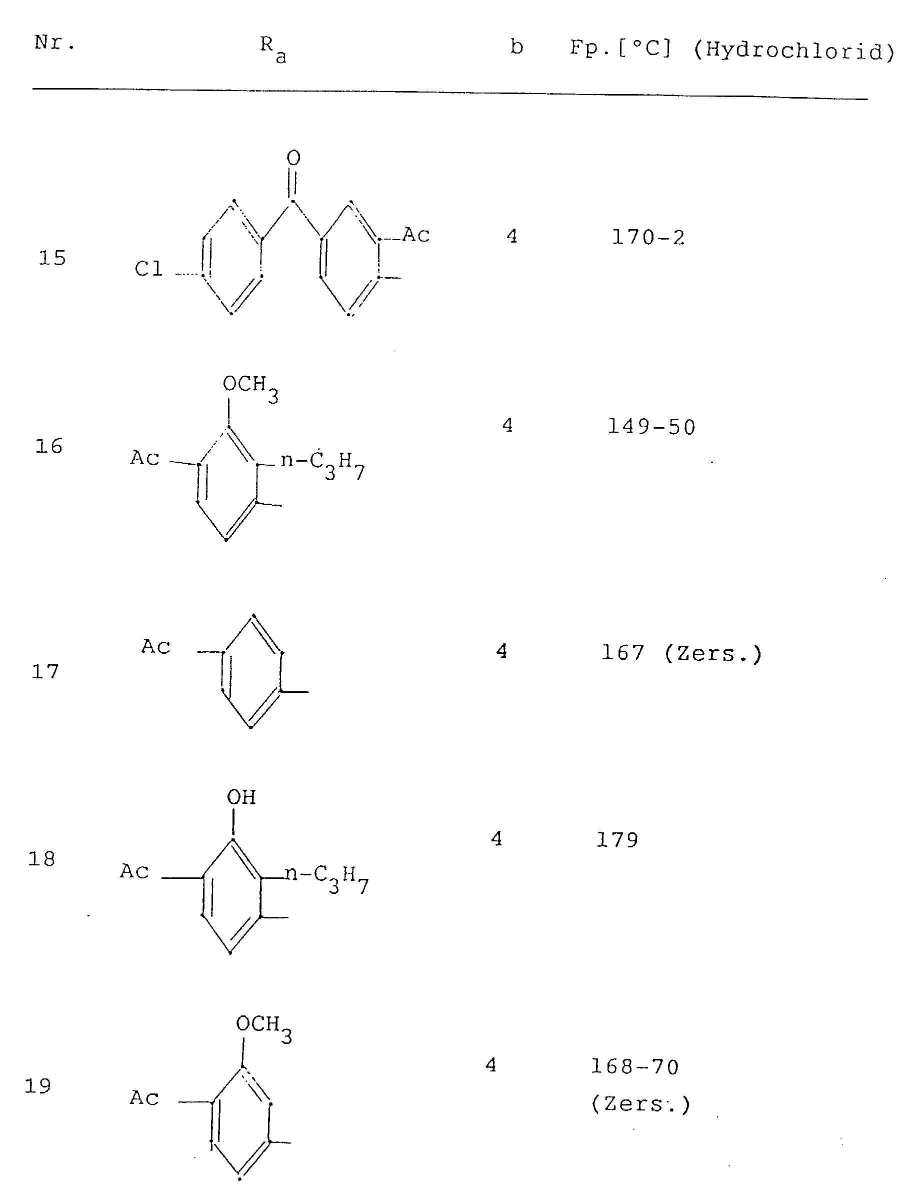

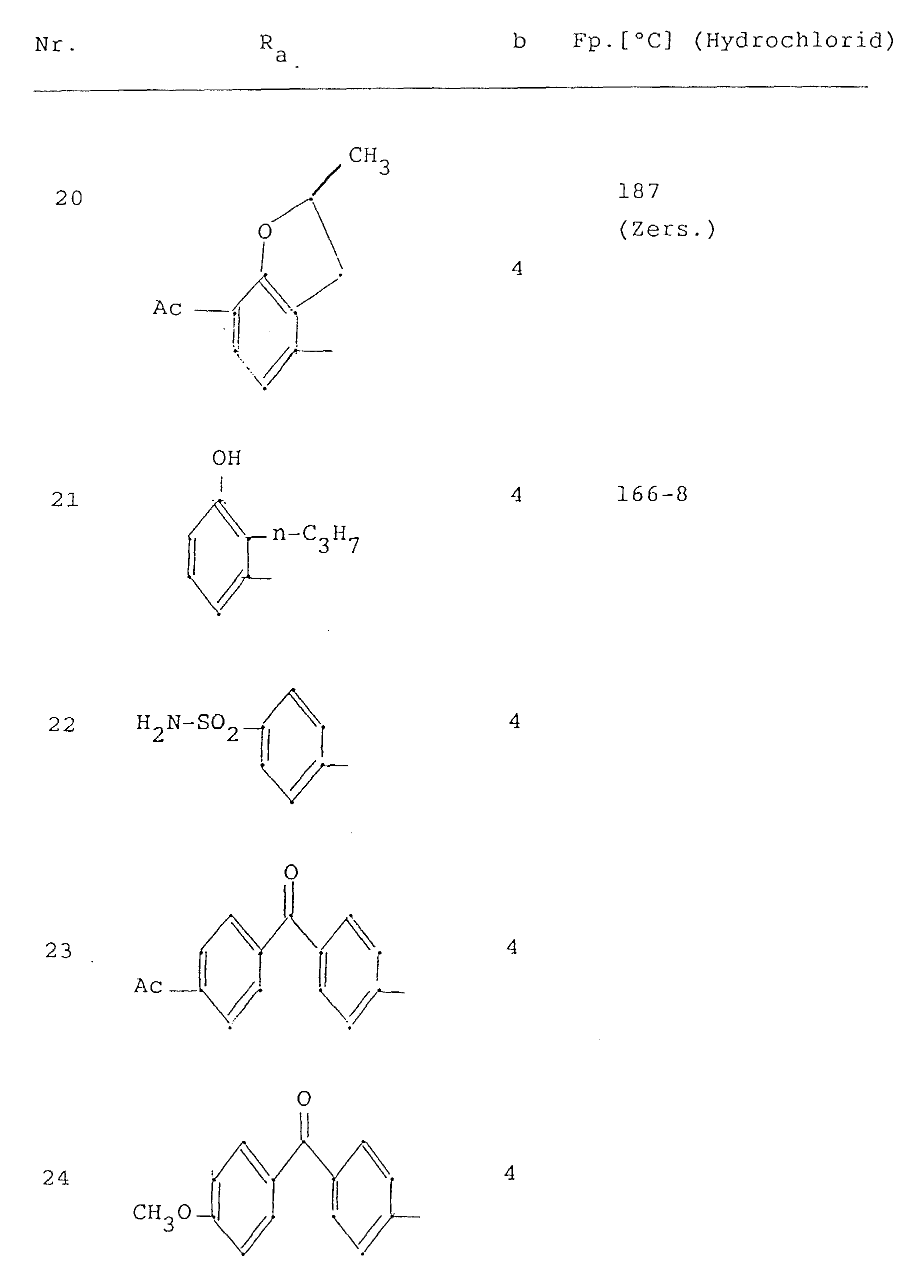

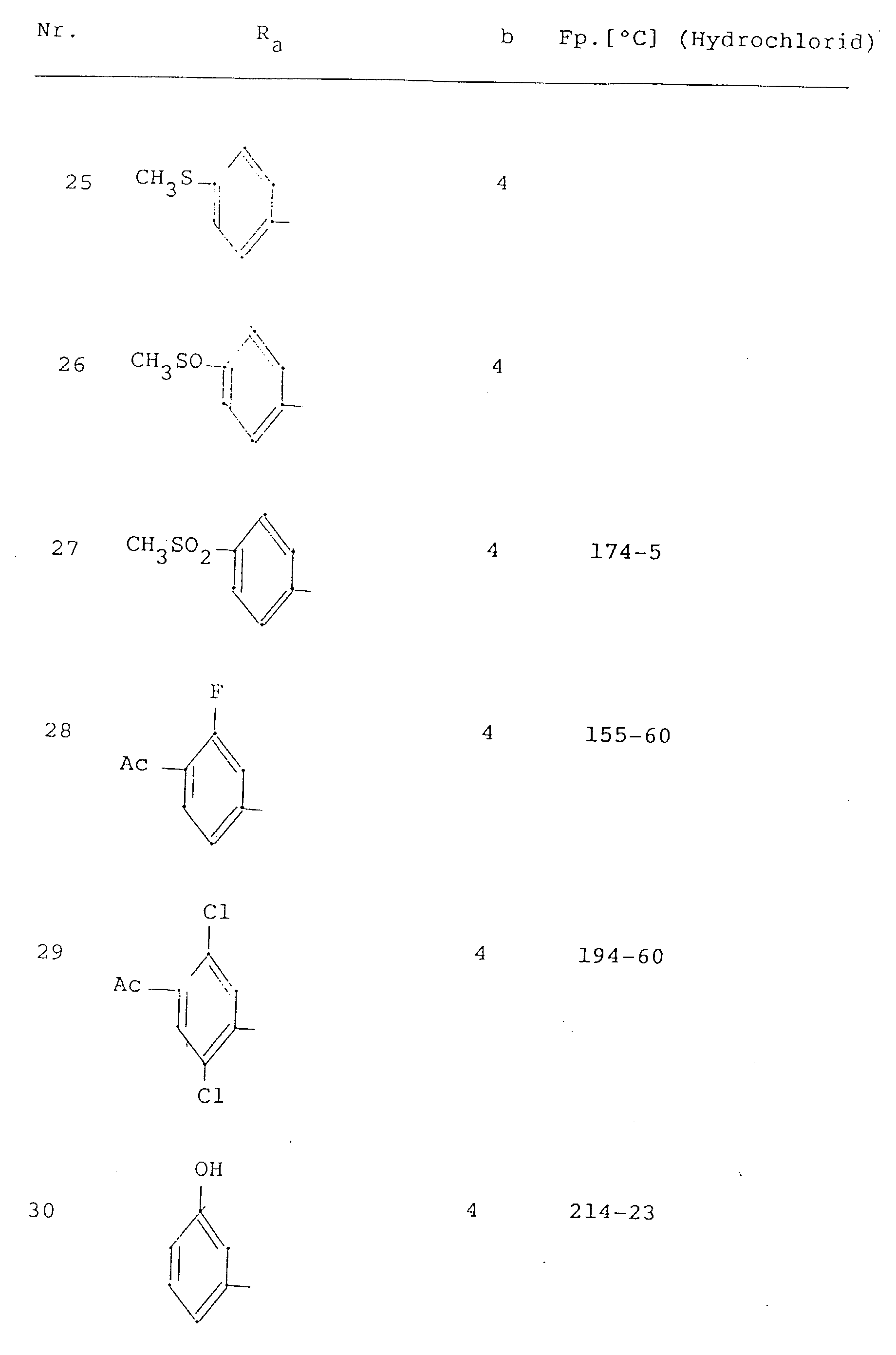

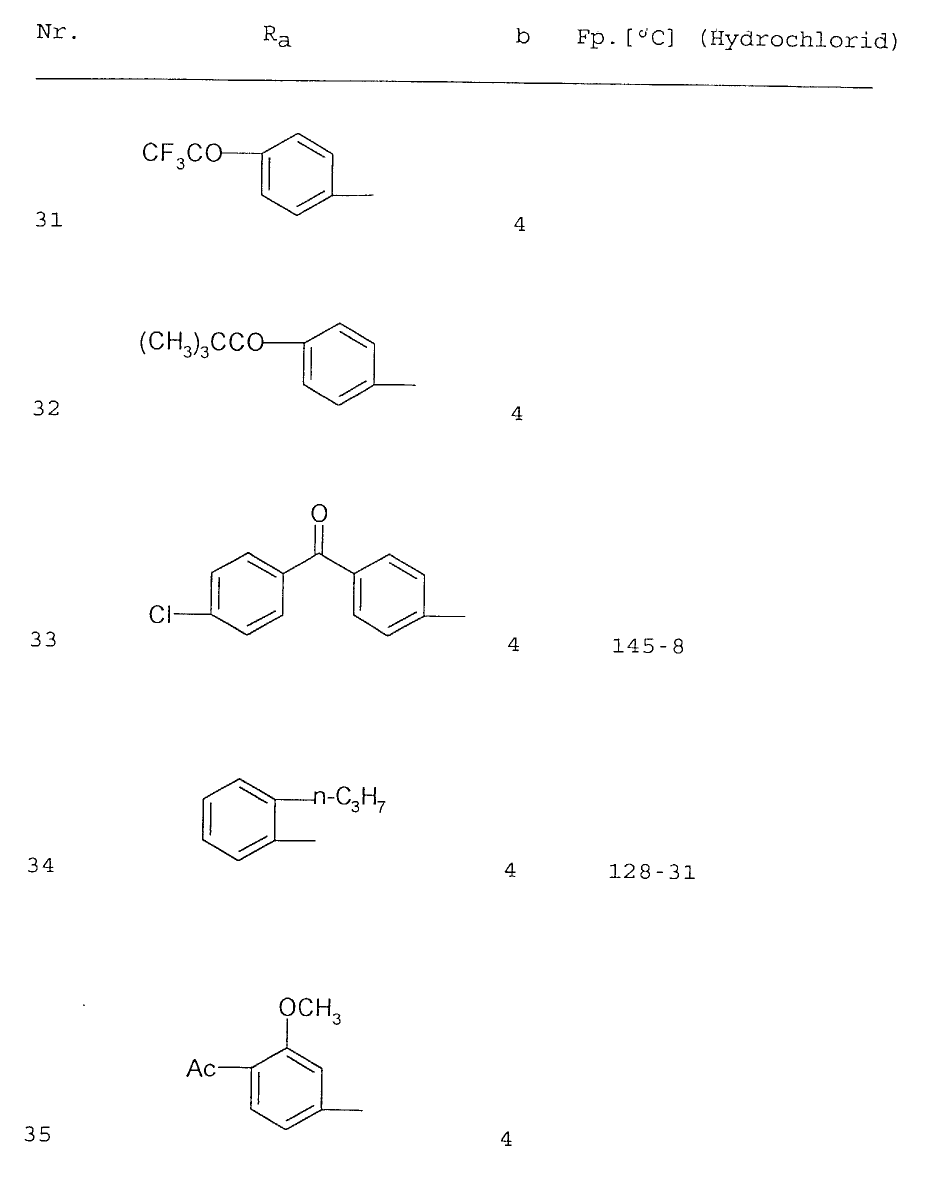

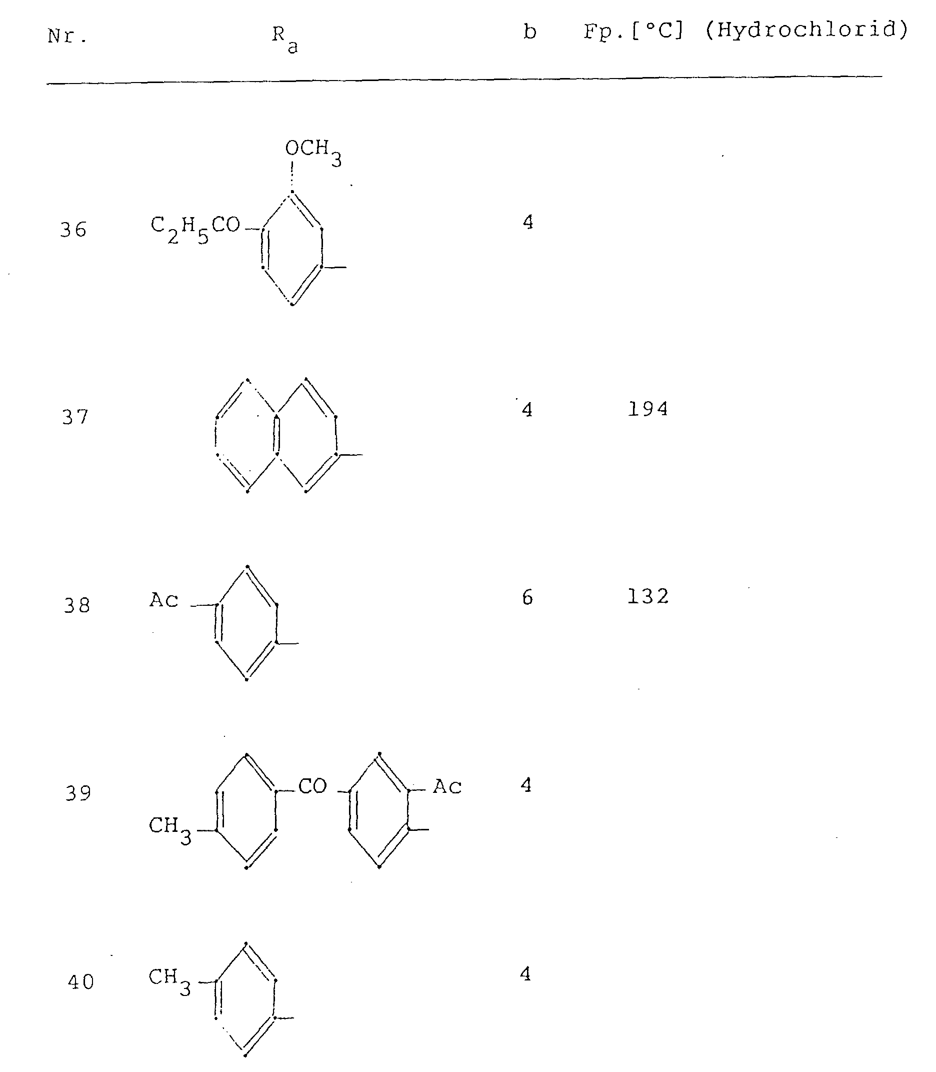

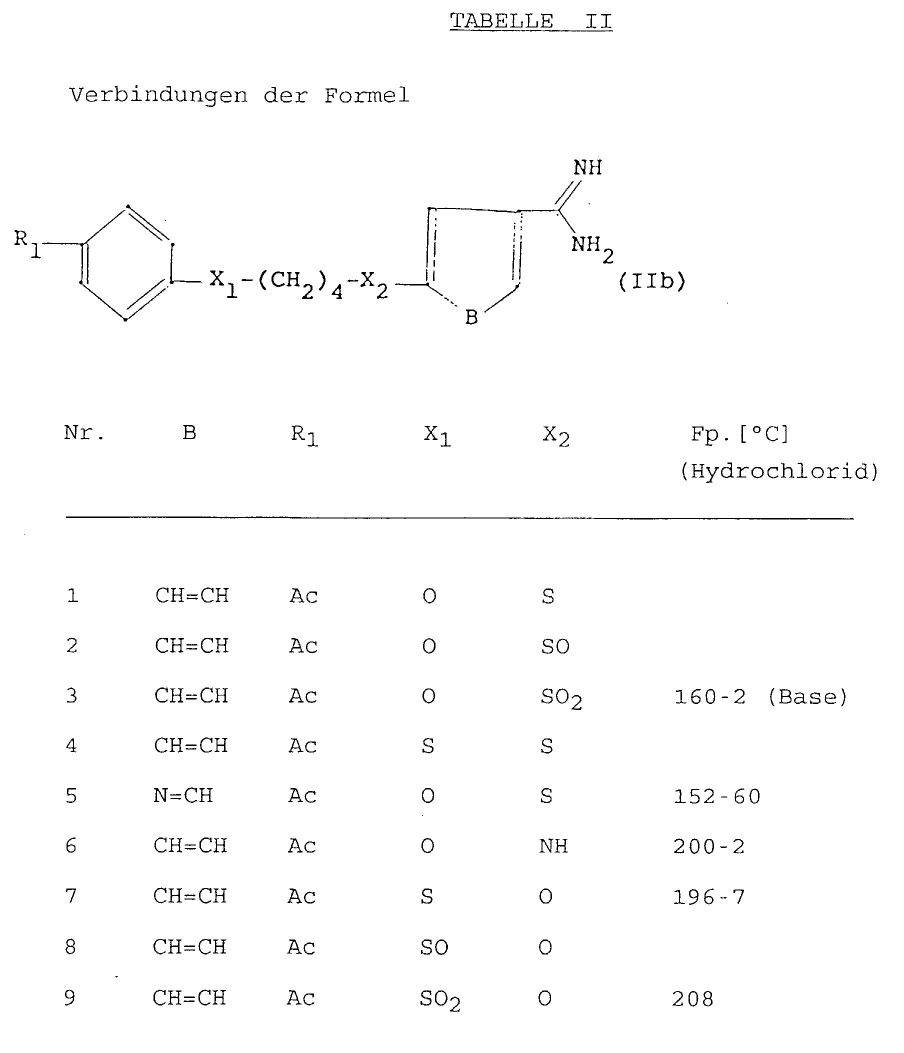

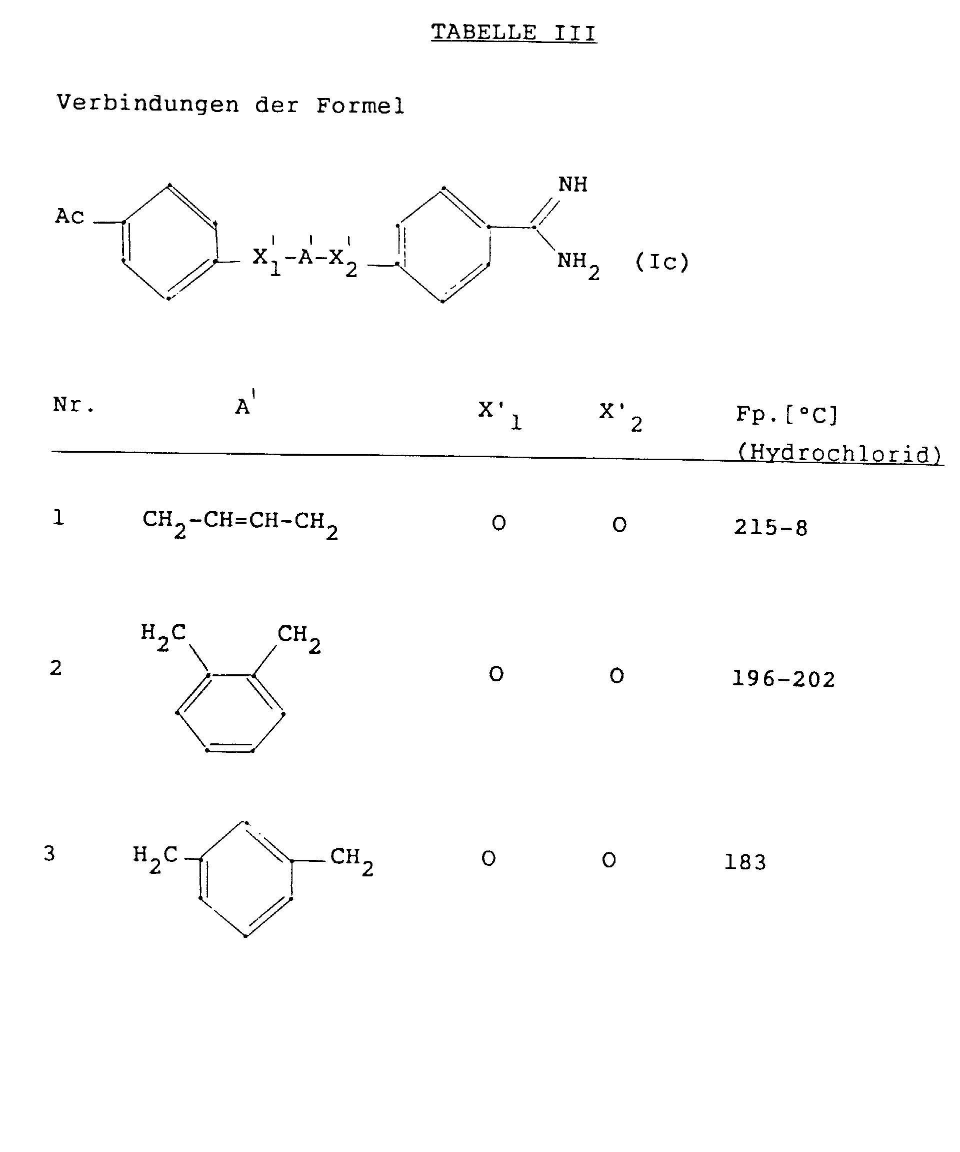

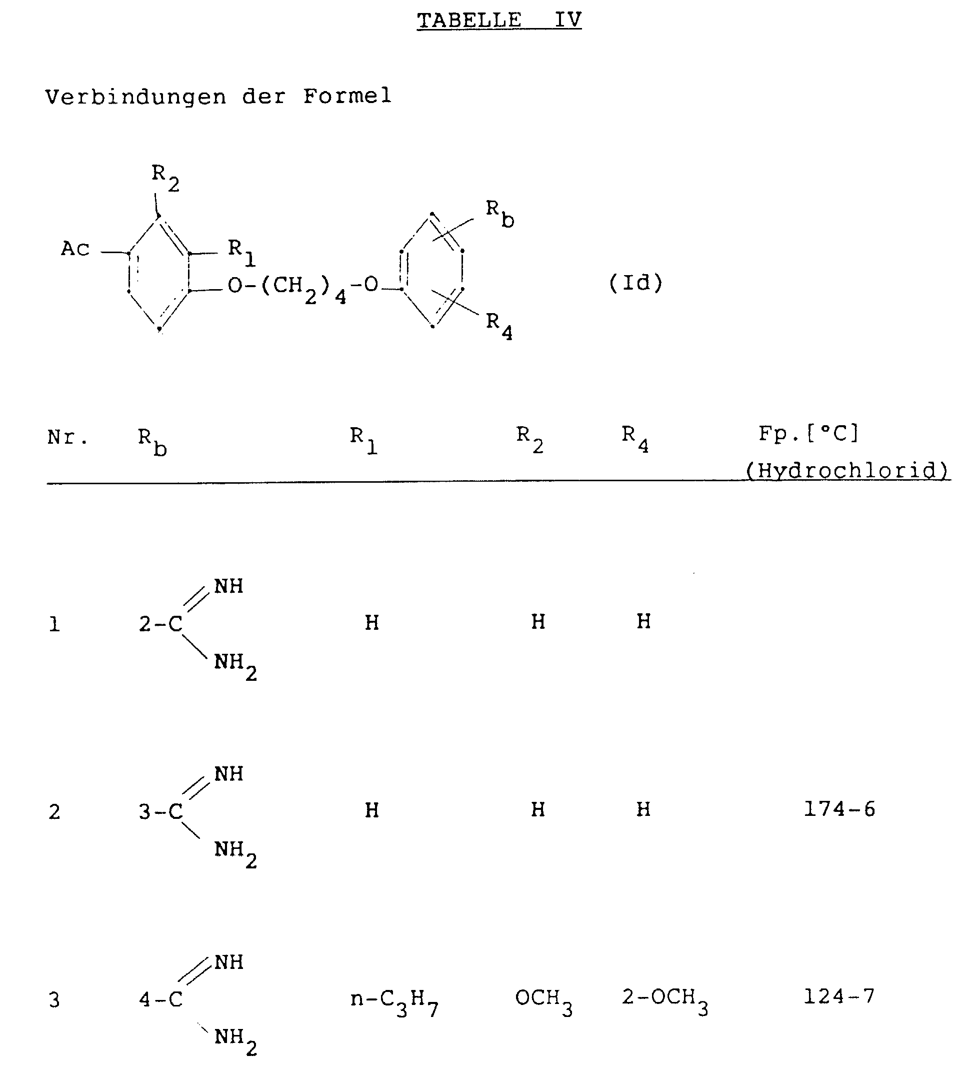

## Patentansprüche

1. Verbindungen der Formel 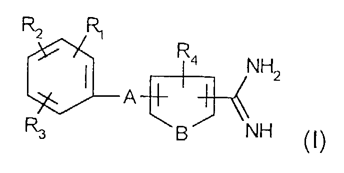 in der
R₁ und R₂, die gleich oder verschieden sein können, für CF₃, Halogen, R₅, OR₅, COR₆, SR₆, SOR₆, SO₂R₆, SO₂NR₅R₇, C(OH)R₅R₇ oder gemeinsam auch für die mit benachbarten C-Atomen des Benzolrings verknüpften zweibindigen Gruppen -CH=CH-CH=CH-, -CR₈=CH-CH=CH-, -CH=CR₈-CH=CH-, -O-CH₂-CH₂-, -O-CH₂-O-, -O-CH₂-CH₂-O-, -(CH₂)₃₋₄-, -NH-CO-O-, -NH-CO-CH₂-O-, -CO-CH₂-O- oder -CO-CH₂CH₂-O- stehen, wobei diese Gruppen ihrerseits auch durch C₁-C₄-Alkyl substituiert sein können,
R₃ für Halogen, OH, CF₃, R₅, OR₆, COR₆, SR₆, SOR₆, SO₂R₆, SO₂NR₅R₇, NR₅R₇ oder C(OH)R₅R₇ steht, wobei, falls R₃ gleich R₅ ist, R₅ nur dann H sein kann, wenn mindestens einer der Substituenten R₁ und R₂ nicht H bedeutet,
R₄ für Wasserstoff, Halogen, OH, C₁-C₄-Alkoxy,
R₅ für H, C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
R₆ für C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
R₇ für H oder C₁-C₁₂-Alkyl,
R₈ für C₂-C₅-Acyl,
A für eine der Gruppen
X₁-A₁-X₂ (II)
X₂-A₂-X₃ (III)
X₄-A₂-X₂ (IV)
(CH₂)₁₋₂-NH-CO-(CH₂)₁₋₃-X₂ (V)
-CH=CH-A₂-X₂ (VI),
B für CH=CH, CH=N, S oder 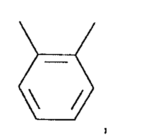
A₁ für C₂-C₄-Alkylen, cis- oder trans-CH₂-CH=CH-CH₂, CH₂-C≡C-CH₂ oder 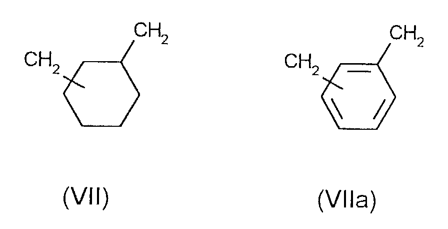
A₂ für C₁-C₅-Alkylen,
X₁ für O, NH, S, SO, SO₂, CO oder CH₂,
X₂ für O, NH oder S,
X₃ für NH-CO, CO-NH oder SO₂-NH,
X₄ für NH-CO, CO-NH, NH-SO₂ oder SO₂-NH steht,
mit der Maßgabe, daß falls
B -CH=CH- und
X₁ Sauerstoff oder Schwefel und
X₂ Sauerstoff oder Schwefel und
A₁ C₂-C₄-Alkylen, cis- oder trans-CH₂-CH≡CH-CH₂, CH₂-C=C-CH₂ oder 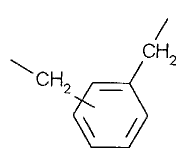 und einer der Reste R₁, R₂ oder R₃ -O-C₁-C₁₂-Alkyl bedeutet,
mindestens keiner der anderen Reste R₁, R₂ oder R₃ nicht
H, Halogen, CF₃, OH, C₁-C₁₂-Alkyl oder -O-C₁-C₁₂-Alkyl sein kann, sowie
mit der Maßgabe, daß falls
B -CH=CH-
R₄ Wasserstoff,
X₁ und X₂ Sauerstoff,
A₁ C₃- oder C₄-Alkylen
bedeuten und die Amidino-Gruppe zum Rest A in meta-Position steht,
R₁, R₂ oder R₃ nicht Phenyl NH₂, Chlor, Methoxy oder CF₃ bedeuten können,
als Racemate, in enantiomerenreiner bzw. angereicherter Form, gegebenenfalls als Diastereomerenpaare sowie in cis- oder trans-Form und jeweils als freie Basen oder als Salze, vorzugsweise mit physiologisch verträglichen Säuren.

2. Verbindungen gemäß Anspruch 1 der allgemeinen Formel 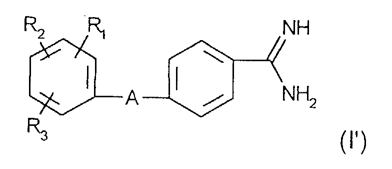 worin
R₁, R₂, die gleich oder verschieden sein können, R₇, OR₇, COR₆ oder gemeinsam auch die mit benachbarten C-Atomen des Benzolrings verknüpften zweibindigen Gruppen-CR₈=CH-CH=CH-, -CH=CR₈-CH=CH-, -O-CH₂-CH₂-, oder -CO-CH₂-CH₂-O- bedeuten, wobei diese Gruppen ihrerseits auch durch C₁-C₄-Alkyl substituiert sein können,
R₃ für Halogen, OH, CF₃, R₅, OR₆, COR₆, SR₆, SOR₆, SO₂R₆, SO₂NR₅R₇, NR₅R₇ oder C(OH)R₅R₇ steht, wobei, falls R₃ gleich R₅ ist, R₅ nur dann H sein kann, wenn mindestens einer der Substituenten R₁ und R₂ nicht H bedeutet,
R₅ für H, C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
R₆ für C₁-C₁₂-Alkyl, Phenyl oder gegebenenfalls durch Halogen, C₁-C₄-Alkyl, C₁-C₄-Alkoxy oder C₂-C₅-Acyl substituiertes Phenyl,
R₇ für H oder C₁-C₁₂-Alkyl,
R₈ für C₂-C₅-Acyl,
und
A für die Gruppe X₁-A₁-X₂ (II) steht, wobei
A₁ C₂-C₄-Alkylen, cis- oder trans-CH₂-CH=CH-CH₂, CH₂-C≡C-CH₂ oder 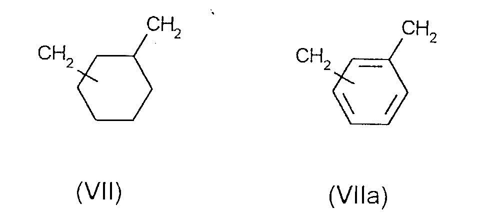
X₁ O, NH, S, SO, SO₂, CO oder CH₂,
X₂ O, NH oder S bedeutet,
mit der Maßgabe, daß falls
X₁ Sauerstoff oder Schwefel und
X₂ Sauerstoff oder Schwefel und
A₁ C₂-C₄-Alkylen, cis- oder trans-CH₂-CH=CH-CH₂, CH₂-C≡C-CH₂ oder 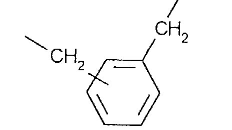 und einer der Reste R₁, R₂ oder R₃ -O-C₁-C₁₂-Alkyl bedeutet,
mindestens keiner der anderen Reste R₁, R₂ oder R₃ nicht
H, Halogen, CF₃, OH, C₁-C₁₂-Alkyl oder -O-C₁-C₁₂-Alkyl sein kann,
als Racemate, in enantiomerenreiner bzw. angereicherter Form, gegebenenfalls als Diastereomerenpaare sowie in cis- oder trans-Form und jeweils als freie Basen oder als Salze, vorzugsweise mit physiologisch verträglichen Säuren.

3. Verbindungen nach Anspruch 1 oder 2, in denen R₁/R₂/R₃ die Bedeutungen C₂-C₅-Acyl/H/H;
C₆H₅CO/H/H; C₁-C₄-Alkyl/OH/H;
C₂-C₅-Acyl/C₁-C₄-Alkyl/H;
C₂-C₅-Acyl/OH/C₁-C₄-Alkyl;
OH/C₂-C₅-Acyl/C₁-C₄-Alkyl besitzen.

4. Verbindungen nach Anspruch 1, 2 und 3, dadurch gekennzeichnet, daß A
für O-(CH₂)₂-O, O-(CH₂)₄-O oder 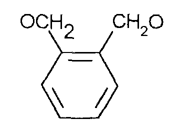 steht.

5. Verbindungen nach Anspruch 1, 2, 3 oder 4, dadurch gekennzeichnet, daß die Gruppe 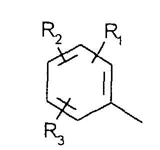 Acetylphenyl ist.

6. Verbindungen nach Anspruch 1, 3, 4 oder 5, dadurch gekennzeichnet, daß die Gruppe 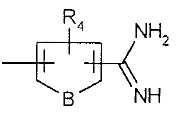 für 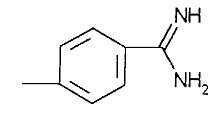 steht.

7. Arzneimittel, gekennzeichnet durch einen Gehalt an einer Verbindung nach Anspruch 1 bis 6.

8. Verwendung von Verbindungen nach Anspruch 1 bis 6 zur Herstellung von Arzneimitteln für die Behandlung von Krankheiten, bei denen entzündliche und/oder allergische Vorgänge eine Rolle spielen, insbesondere Asthma, Colitis ulcerosa, Psoriasis und zur Behandlung einer durch nichtsteroidale Antiphlogistika induzierten Gastropathie.

9. Verfahren zur Herstellung von Verbindungen nach Anspruch 1 bis 6, dadurch gekennzeichnet, daß man
a) einen Imidoester der Formel 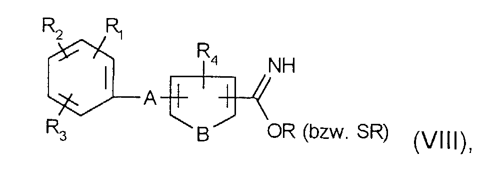 in der R₁ bis R₄, A und B die obige Bedeutung haben und R bevorzugt für einen C₁-C₆-Alkylrest oder für Benzyl steht, mit Ammoniak umsetzt oder daß man
b1) ein Phenol oder Thiophenol der Formel 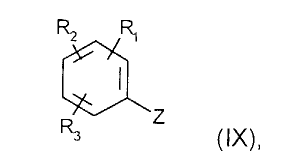 worin Z OH oder SH darstellt und R₁, R₂ und R₃ die obige Bedeutung haben, mit einer Verbindung der Formel 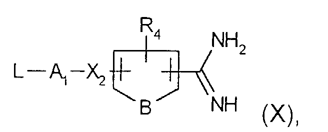 bzw. 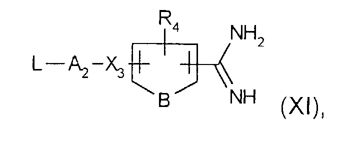 worin A₁, A₂, B, R₄, X₂ und X₃ die obige Bedeutung haben und L für eine nucleofuge Abgangsgruppe steht, bzw.
(b2) ein Phenol oder Thiophenol der Formel 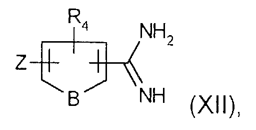 worin B, R₄ und Z die obige Bedeutung haben, mit einer Verbindung der Formel 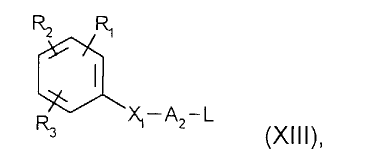 bzw. 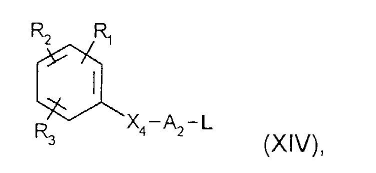 bzw. 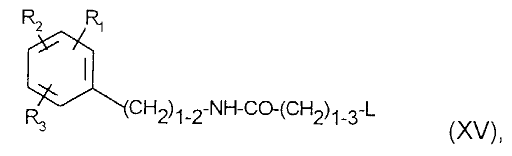 bzw. 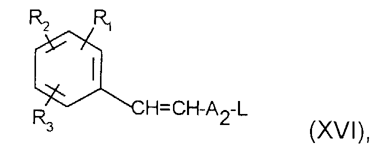 worin A₁, A₂, R₁, R₂, R₃ und L die obige Bedeutung haben, umsetzt, oder daß man
c) ein Amidoxim der Formel 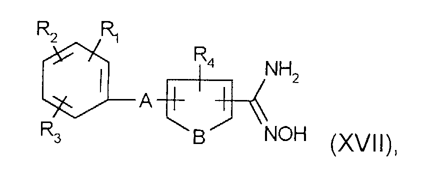 worin A, B, und R₁ bis R₄ die obige Bedeutung haben, zu dem entsprechenden Amidin reduziert.

## Claims

1. Compounds of the formula 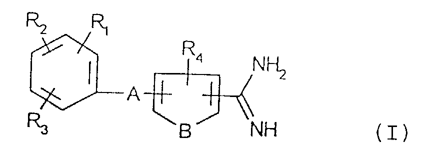 wherein
R₁ and R₂, which may be identical or different, denote CF₃, halogen, R₅, OR₅, COR₆, SR₆, SOR₆, SO₂R₆, SO₂NR₅R₇, C(OH)R₅R₇ or together may also denote the double-bonded groups -CH=CH-CH=CH-, -CR₈=CH-CH=CH-, -CH=CR₈-CH=CH-, -O-CH₂-CH₂-, -O-CH₂-O-, -O-CH₂-CH₂-O-, -(CH₂)₃₋₄-, -NH-CO-O-, -NH-CO-CH₂-O-, -CO-CH₂-O- or -CO-CH₂CH₂-O-, linked with adjacent carbon atoms of the benzene ring, whilst these groups may in turn be substituted by C₁₋₄-alkyl,
R₃ denotes halogen, OH, CF₃, R₅, OR₆, COR₆, SR₆, SOR₆, SO₂R₆, SO₂NR₅R₇, NR₅R₇ or C(OH)R₅R₇ (whilst if R₃ is the same as R₅, R₅ can only denote H if at least one of the substituents R₁ and R₂ does not denote H),
R₄ denotes hydrogen, halogen, OH, C₁₋₄-alkoxy,
R₅ denotes H, C₁₋₁₂-alkyl, phenyl, phenyl optionally substituted by halogen, C₁₋₄-alkyl, C₁₋₄-alkoxy or C₂₋₅-acyl,
R₆ denotes C₁₋₁₂-alkyl, phenyl, or phenyl optionally substituted by halogen, C₁₋₄-alkyl, C₁₋₄-alkoxy or C₂₋₅-acyl,
R₇ denotes H or C₁₋₁₂-alkyl,
R₈ denotes C₂₋₅-acyl,
A denotes one of the groups
X₁-A₁-X₂ (II)
X₂-A₂-X₃ (III)
X₄-A₂-X₂ (IV)
(CH₂)₁₋₂-NH-CO-(CH₂)₁₋₃-X₂ (V)
-CH=CH-A₂-X₂ (VI)
B denotes CH=CH, CH=N, S or 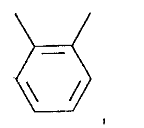
A₁ denotes C₂₋₄-alkylene, cis- or trans-CH₂-CH=CH-CH₂, CH₂-C≡C-CH₂ or 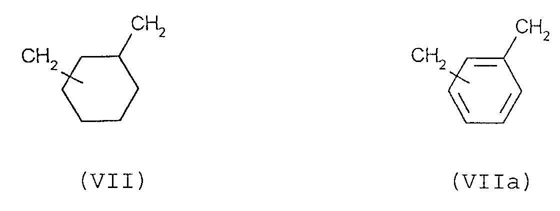
A₂ denotes C₁₋₅-alkylene,
X₁ denotes O, NH, S, SO, SO₂, CO or CH₂,
X₂ denotes O, NH or S,
X₃ denotes NH-CO, CO-NH or SO₂-NH,
X₄ denotes NH-CO, CO-NH, NH-SO₂ or SO₂-NH,
with the proviso that if
B denotes -CH=CH- and
X₁ denotes oxygen or sulphur and
X₂ denotes oxygen or sulphur and
A₁ denotes C₂₋₄-alkylene, cis- or trans-CH₂-CH=CH-CH₂, CH₂-C≡C-CH₂ or 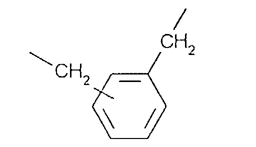 and one of the groups R₁, R₂ or R₃ denotes -O-C₁₋₁₂-alkyl,
at least one of the other groups R₁, R₂ or R₃ cannot be hydrogen, halogen, CF₃, OH, C₁₋₁₂-alkyl or -O-C₁₋₁₂-alkyl,
and with the proviso that if
B denotes -CH=CH-
R₄ denotes hydrogen,
X₁ and X₂ denote oxygen,
A₁ denotes C₃- or C₄-alkylene
and the amidino group is in the meta position to the group A,
R₁, R₂ or R₃ cannot denote phenyl, NH₂, chlorine, methoxy or CF₃,
as racemates, in enantiomerically pure or concentrated form, possibly as pairs of diastereomers and in cis- or trans-form and as free bases or as salts, preferably with physiologically acceptable acids.

2. Compounds according to claim 1 of general formula 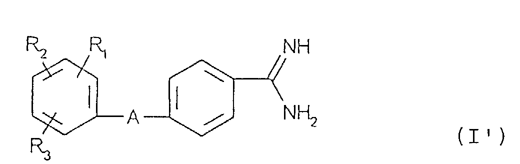 wherein
R₁, R₂, which may be identical or different, denote R₇, OR₇, COR₆, or together denote the double bonded groups-CR₈=CH-CH=CH-,-CH=CR₈-CH=CH-, -O-CH₂-CH₂- or -CO-CH₂-CH₂-O-, linked with adjacent carbon atoms of the benzene ring, whilst these groups may in turn be substituted by C₁₋₄-alkyl,
R₃ denotes halogen, OH, CF₃, R₅, OR₆, COR₆, SR₆, SOR₆, SO₂R₆, SO₂NR₅R₇, NR₅R₇ or C(OH)R₅R₇ (whilst if R₃ is equal to R₅, R₅ can only denote H if at least one of the substituents R₁ and R₂ does not denote H),
R₅ denotes H, C₁₋₂-alkyl, phenyl, phenyl optionally substituted by halogen, C₁₋₄-alkyl, C₁₋₄-alkoxy or C₂₋₅-acyl,
R₆ denotes C₁₋₁₂-alkyl, phenyl, or phenyl optionally substituted by halogen, C₁₋₄-alkyl, C₁₋₄-alkoxy or C₂₋₅-acyl,
R₇ denotes H or C₁₋₁₂-alkyl,
R₈ denotes C₂₋₅-acyl,
and
A denotes the group X₁-A₁-X₂ (II) wherein
A₁ denotes C₂₋₄-alkylene, cis- or trans-CH₂-CH≡CH-CH₂, CH₂-C≡C-CH₂ or 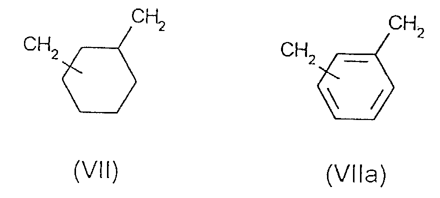
X₁ denotes O, NH, S, SO, SO₂, CO or CH₂,
X₂ denotes O, NH or S,
with the proviso that if
X₁ denotes oxygen or sulphur and
X₂ denotes oxygen or sulphur and
A₁ denotes C₂₋₄-alkylene, cis- or trans-CH₂-CH=CH-CH₂, CH₂-C≡C-CH₂ or 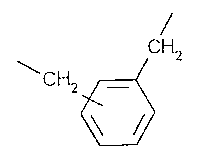 and
one of the groups R₁, R₂ or R₃ denotes -O-C₁₋₁₂-alkyl, at least one of the other groups R₁, R₂ or R₃ cannot denote hydrogen, halogen, CF₃, OH, C₁₋₁₂-alkyl or -O-C₁₋₁₂-alkyl,
as racemates, in enantiomerically pure or concentrated form, optionally as pairs of diastereomers and in cis or trans form and as free bases or salts, preferably with physiologically acceptable acids.

3. Compounds according to claim 1 or 2, wherein R₁/R₂/R₃ have the meanings C₂₋₅-acyl/H/H;
C₆H₅CO/H/H; C₁₋₄-alkyl/OH/H;
C₂₋₅-acyl/C₁₋₄-alkyl/H;
C₂₋₅-acyl/oH/C₁₋₄-alkyl;
OH/C₂₋₅-acyl/C₁₋₄-alkyl.

4. Compounds according to claim 1, 2 and 3, characterised in that A denotes
O-(CH₂)₂-O, O-(CH₂)₄-O or

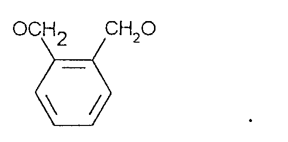

5. Compounds according to-claim 1, 2, 3 or 4, characterised in that the group 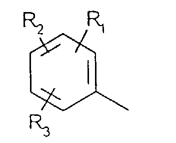 is acetylphenyl.

6. Compounds according to claim 1, 3, 4 or 5, characterised in that the group 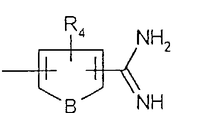 represents

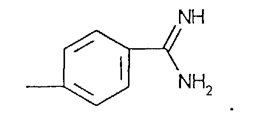

7. Pharmaceutical compositions, characterised in that they contain a compound according to claims 1 to 6.

8. Use of compounds according to claims 1 to 6 in the preparation of pharmaceutical compositions for the treatment of diseases in which inflammatory and/or allergic processes are involved, especially asthma, ulcerative colitis, psoriasis and for treating gastropathy induced by non-steroidal antiphlogistics.

9. Process for preparing compounds according to claims 1 to 6, characterised in that
a) an imidoester of the formula 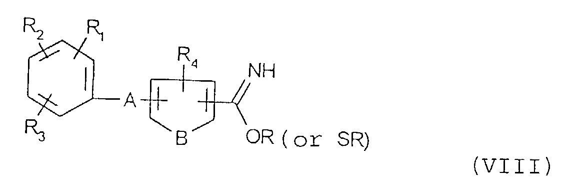 wherein R₁ to R₄, A and B are as hereinbefore defined and R preferably represents a C₁₋₆-alkyl group or benzyl, is reacted with ammonia, or
b1) a phenol or thiophenol of the formula 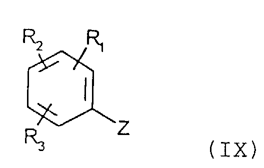 wherein Z denotes OH or SH and R₁, R₂ and R₃ are as hereinbefore defined, is reacted with a compound of formula 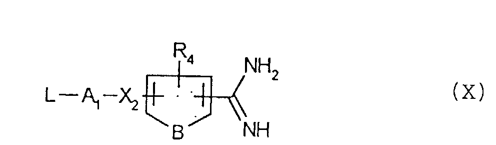 or 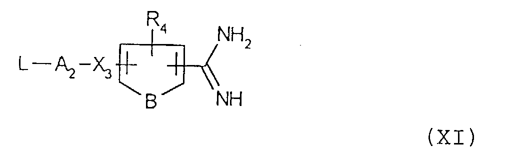 wherein A₁, A₂, B, R₄, X₂ and X₃ are as hereinbefore defined and L denotes a nucleofugic leaving group, or 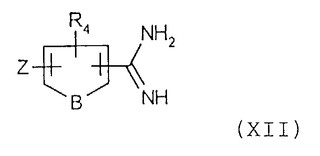 wherein B, R₄ and Z are as hereinbefore defined, is reacted with a compound of formula 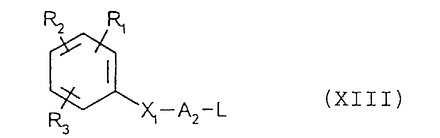 or 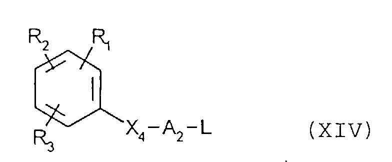 or 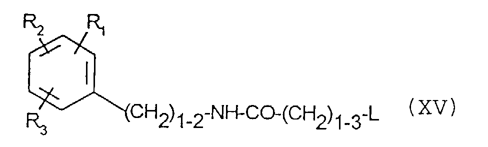 or 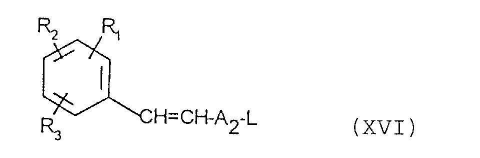 wherein A₁, A₂, R₁, R₂, R₃ and L are as hereinbefore defined; or
c) an amidoxime of the formula 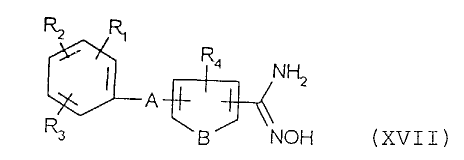 wherein A, B and R₁ to R₄ are as hereinbefore defined, is reduced to form the corresponding amidine.

## Revendications

1. Composés de formule 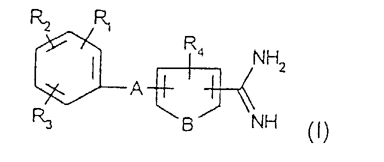 dans laquelle
R₁ et R₂, qui peuvent être identiques ou différents, représentent CF₃, halogène, R₅, OR₅, COR₆, SR₆, SOR₆, SO₂R₆, SO₂NR₅R₇, C(OH)R₅R₇ ou encore, ensemble, les groupes divalents liés à des atomes C voisins du cycle benzénique -CH=CH-CH=CH-, -CR₈=CH-CH=CH-, -CH=CR₈-CH=CH-, -O-CH₂-CH₂-, -O-CH₂-O-, -O-CH₂-CH₂-O-, -(CH₂)₃₋₄-, -NH-CO-O-, -NH-CO-CH₂-O-, -CO-CH₂-O- ou -CO-CH₂CH₂-O-, ces groupes pouvant pour leur part être substitués aussi par alkyle en C₁-C₄,
R₃ représente halogène, OH, CF₃, R₅, OR₆, COR₆, SR₆, SOR₆, SO₂R₆, SO₂NR₅R₇, NR₅R₇ ou C(OH)R₅R₇ où, au cas où R₃ est R₅, R₅ ne peut être H que si au moins l'un des substituants R₁ et R₂ ne représente pas H,
R₄ représente hydrogène, halogène, OH, alcoxy en C₁-C₄,
R₅ représente H, alkyle en C₁-C₁₂, phényle, phényle éventuellement substitué par halogène, alkyle en C₁-C₄, alcoxy en C₁-C₄ ou acyle en C₂-C₅,
R₆ représente alkyle en C₁-C₁₂, phényle ou phényle éventuellement substitué par halogène, alkyle en C₁-C₄, alcoxy en C₁-C₄ ou acyle en C₂-C₅,
R₇ représente H ou alkyle en C₁-C₁₂,
R₈ représente acyle en C₂-C₅,
A représente l'un des groupes
X₁-A₁-X₂ (II)
X₂-A₂-X₃ (III)
X₄-A₂-X₂ (IV)
(CH₂)₁₋₂-NH-CO-(CH₂)₁₋₃-X₂ (V)
-CH=CH-A₂-X₂ (VI)
B représente CH=CH, CH=N, S ou 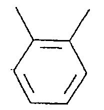
A₁ représente alkylène en C₂-C₄, cis- ou trans-CH₂-CH=CH-CH₂, CH₂-C≡C-CH₂ ou 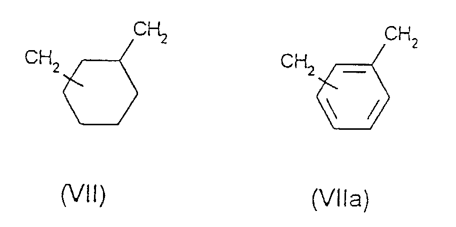
A₂ représente alkylène en C₁-C₅,
X₁ représente O, NH, S, SO, SO₂, CO ou CH₂,
X₂ représente O, NH ou S,
X₃ représente NH-CO, CO-NH ou SO₂-NH,
X₄ représente NH-CO, CO-NH, NH-SO₂ ou SO₂-NH,
à condition que si
B représente -CH=CH- et
X₁ représente l'oxygène ou le soufre et
X₂ représente l'oxygène ou le soufre et
A₁ représente alkylène en C₂-C₄, cis- ou trans-CH₂-CH=CH-CH₂, CH₂-C≡C-CH₂ ou 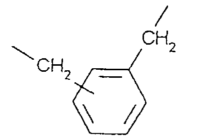 et
l'un des restes R₁, R₂ et R₃ représente -O-alkyle en C₁-C₁₂, au moins l'un des autres restes R₁, R₂ et R₃ ne peut pas représenter hydrogène, halogène, CF₃, OH, alkyle en C₁-C₁₂ ou -O-alkyle en C₁-C₁₂,
et à condition que si
B représente -CH=CH-
R₄ représente l'hydrogène,
X₁ et X₂ représentent l'oxygène,
A₁ représente alkylène en C₃ ou C₄
et le groupe amidino est en position méta par rapport au reste A,
R₁, R₂ ou R₃ ne peut pas représenter phényle, NH₂, chlore, méthoxy ou CF₃,
sous forme de racémates, sous forme énantiomériquement pure ou enrichie, éventuellement sous forme de paires de diastéréoisomères ainsi que sous forme cis ou trans et dans chaque cas sous forme de bases libres ou de sels, de préférence avec des acides physiologiquement acceptables.

2. Composés selon la revendication 1 de formule générale 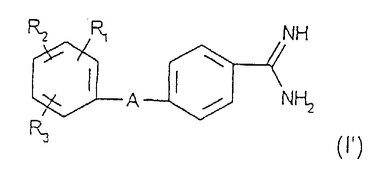 dans laquelle
R₁ et R₂, qui peuvent être identiques ou différents, représentent R₇, OR₇, COR₆ ou encore, ensemble, les groupes divalents liés à des atomes C voisins du cycle benzénique -CR₈=CH-CH=CH-, -CH=CR₈-CH=CH-, -O-CH₂-CH₂- ou -CO-CH₂-CH₂-O-, ces groupes pouvant pour leur part être substitués aussi par alkyle en C₁-C₄,
R₃ représente halogène, OH, CF₃, R₅, OR₆, COR₆, SR₆, SOR₆, SO₂R₆, SO₂NR₅R₇, NR₅R₇ ou C(OH)R₅R₇ où, si R₃ est R₅, R₅ ne peut être H que si au moins l'un des substituants R₁ et R₂ ne représente pas H,
R₅ représente H, alkyle en C₁-C₁₂, phényle ou phényle éventuellement substitué par halogène, alkyle en C₁-C₄, alcoxy en C₁-C₄ ou acyle en C₂-C₅,
R₆ représente alkyle en C₁-C₁₂, phényle ou phényle éventuellement substitué par halogène, alkyle en C₁-C₄, alcoxy en C₁-C₄ ou acyle en C₂-C₅,
R₇ représente H ou alkyle en C₁-C₁₂,
R₈ représente acyle en C₂-C₅
et
A représente le groupe X₁-A₁-X₂ (II), où
A₁ représente alkylène en C₂-C₄, cis- ou trans-CH₂-CH=CH-CH₂, CH₂-C≡C-CH₂ ou 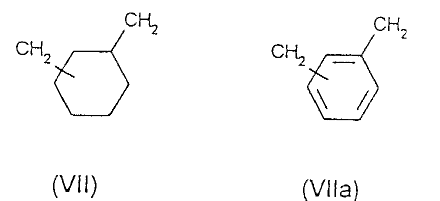
X₁ représente O, NH, S, SO, SO₂, CO ou CH₂,
X₂ représente O, NH ou S,
à condition que si
X₁ représente l'oxygène ou le soufre et
X₂ représente l'oxygène ou le soufre et
A₁ représente alkylène en C₂-C₄, cis- ou trans-CH₂-CH=CH-CH₂, CH₂-C≡C-CH₂ ou 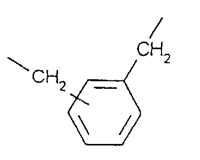
et
l'un des restes R₁, R₂ et R₃ représente -O-alkyle en C₁-C₁₂, au moins l'un des autres restes R₁, R₂ et R₃ ne peut pas représenter H, halogène, CF₃, OH, alkyle en C₁-C₁₂ ou -O-alkyle en C₁-C₁₂,
sous forme de racémates, sous forme énantiomériquement pure ou enrichie, éventuellement sous forme de paires de diastéréoisomères ainsi que sous forme cis ou trans et dans chaque cas sous forme de bases libres ou de sels, de préférence avec des acides physiologiquement acceptables.

3. Composés selon la revendication 1 ou 2 dans lesquels
R₁/R₂/R₃ possèdent les significations de acyle en C₂-C₅/H/H, C₆H₅CO/H/H, alkyle en C₁-C₄/OH/H,
acyle en C₂-C₅/alkyle en C₁-C₄/H,
acyle en C₂-C₅/OH/alkyle en C₁-C₄,
OH/acyle en C₂-C₅/alkyle en C₁-C₄.

4. Composés selon les revendications 1, 2 et 3 caractérisés en ce que A représente
O-(CH₂)₂-O, O-(CH₂)₄-O ou

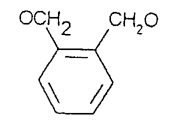

5. Composés selon la revendication 1, 2, 3 ou 4 caractérisés en ce que le groupe 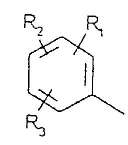 est acétylphényle.

6. Composés selon la revendication 1, 3, 4 ou 5 caractérisés en ce que le groupe 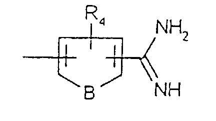 représente

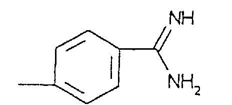

7. Médicament caractérisé en ce qu'il contient un composé selon les revendications 1 à 6.

8. Utilisation de composés selon les revendications 1 à 6 pour la préparation de médicaments pour le traitement de maladies dans lesquelles des processus inflammatoires et/ou allergiques jouent un rôle, en particulier l'asthme, la colite ulcéreuse, le psoriasis et pour le traitement d'une gastropathie induite par des antiphlogistiques non stéroïdes.

9. Procédé pour la préparation de composés selon les revendications 1 à 6 caractérisé en ce que l'on fait réagir
a) un imidoester de formule 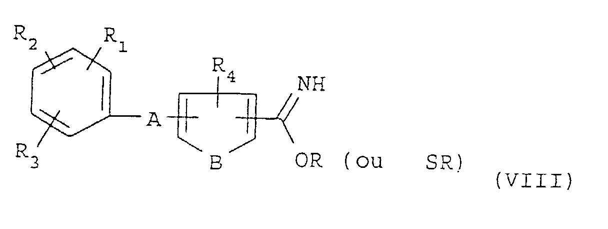 dans laquelle R₁ à R₄, A et B ont la signification ci-dessus et R représente de préférence un reste alkyle en C₁-C₆ ou benzyle avec l'ammoniac, ou bien en ce que l'on fait réagir
(b1) un phénol ou thiophénol de formule 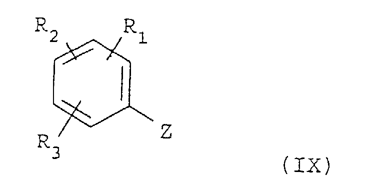 où Z représente OH ou SH et R₁, R₂ et R₃ ont la signification ci-dessus, avec un composé de formule 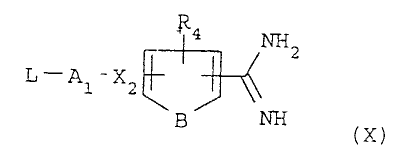 ou 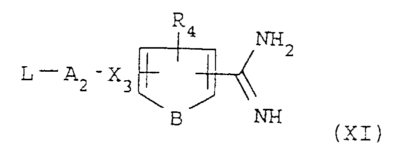 où A₁, A₂, B, R₄, X₂ et X₃ ont la signification ci-dessus et L représente un groupe partant nucléofuge, ou
(b2) un phénol ou thiophénol de formule 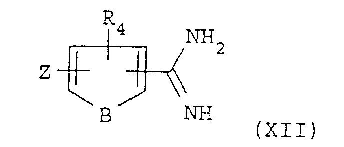 où B, R₄ et Z ont la signification ci-dessus, avec un composé de formule 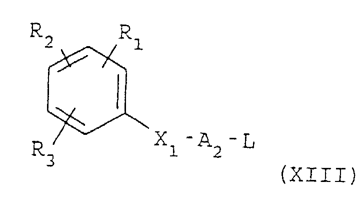 ou 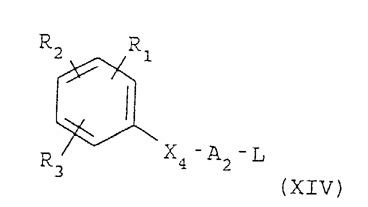 ou 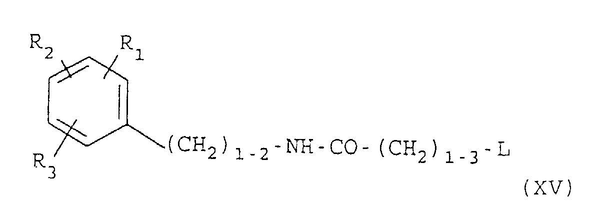 ou 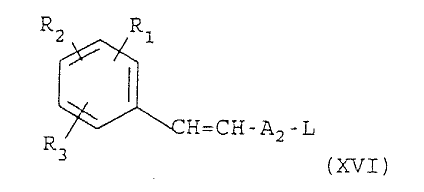 où A₁, A₂, R₁, R₂, R₃ et L ont la signification ci-dessus, ou bien en ce que l'on
c) réduit une amidoxime de formule 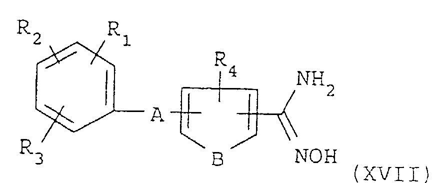 où A, B et R₁ à R₄ ont la signification ci-dessus en l'amidine correspondante.
